# EUROPEAN PATENT SPECIFICATION

(11) **EP 3 773 328 B1**
(45) Date of publication and mention of the grant of the patent: **20.03.2024**
(21) Application number: 19781243.1
(22) Date of filing: 02.04.2019
(51) Int. Cl.: A61F 2/00, A61F 2/52, A61L 27/22, A61L 27/24, A61L 27/36, A61L 27/38

(54) **POLAND SYNDROME AND METHODS OF TREATMENT**
POLEN-SYNDROM UND BEHANDLUNGSVERFAHREN
SYNDROME DE POLAND ET MÉTHODES DE TRAITEMENT

(30) Priority: 02.04.2018 US 201862651447 P
(43) Date of publication of application: 17.02.2021
(73) Proprietor: Bioaesthetics Corporation, Durham, NC 27701 (US)
(72) Inventor: PASHOS, Nicholas, Durham, North Carolina 27703 (US)
(74) Representative: Page White Farrer
(86) International application number: PCT/US2019/025305
(87) International publication number: WO 2019/195226

(56) References cited:
- WO-A1-2017/100713
- US-A1- 2013 280 801
- US-A1- 2018 015 204
- US-A1- 2018 015 204
- N C PASHOS ET AL: "A Tissue Engineered Nipple and Areola Complex", MOLECULAR THERAPY, vol. 23, no. 1, 1 May 2015 (2015-05-01), pages s254-s255, XP055391372, DOI: 10.1016/S1525-0016(16)34249-6
- PATRICK C W: "Tissue Engineering Strategies for Adipose Tissue Repair", THE ANATOMICAL RECORD, A.R. LISS, NEW YORK, NY, US, vol. 263, no. 4, 1 August 2001 (2001-08-01), pages 361-366, XP003021962, ISSN: 0003-276X, DOI: 10.1002/AR.1113

## Description

This application claims priority from U.S. Provisional Patent Application No. 62/651,447, filed on April 2, 2018.

### FIELD OF THE INVENTION

This invention is directed to grafts and compositions comprising decellularized epidermis and/or dermis and essentially a matrix molecule in conjunction with a seeded cell. The invention is further directed to the same grafts and compositions for use to treat Poland Syndrome. The invention is defined by the claims.

### BACKGROUND OF THE INVENTION

Poland Syndrome is a disorder in which individuals are born with missing or underdeveloped muscles on one side of the body, resulting in abnormalities that can affect, for example, the chest. Poland Syndrome is sporadic, and is more common in males vs. females. The cause of Poland Syndrome is unknown. The incidence of the condition ranges from about 1 in 30,000 to about 1 in 70,000.

US 2018/0015204 A1 (Pashos et al., published 18 January 2018), reports "Surgical grafts for replacing the nipple and areola or damaged epidermis". WO 2017/100713 A1 (Novothelium, LLC., published 15 June 2017), reports "Human nipple areolar complex extracellular matrix scaffold and methods related thereto". Pashos et al., reports "A tissue engineered nipple and areola complex" (Molecular Therapy, vol. 23, supplement no. 1, 1 May 2015, pages s254-s255). Patrick, C.W., reports "Tissue engineering strategies for adipose tissue replair" (The Anatomical Record, vol. 263, no. 4, 1 August 2001, pages 361-366).

### SUMMARY OF THE INVENTION

The present invention provides for compositions consisting essentially of decellularized dermis and/or decellularized epidermis according to claim 1. The present invention also provides for an implantable surgical graft according to claim 7, and a decellularized nipple, areola, or nipple attached to an areola for use in grafting to the chest of a subject with Poland Syndrome. Poland Syndrome is a developmental disorder in which patients have underdeveloped chest muscles and may lack NACs.

An aspect of the invention is directed to compositions consisting essentially of decellularized dermis and/or decellularized epidermis, wherein the dermis and/or epidermis retain(s) at least one matrix molecule selected from the group consisting of laminin, elastin, fibronectin, and collagen; and wherein the decellularized dermis and/or decellularized epidermis further comprises exogenous seeded cells comprising a combination of keratinocytes, melanocytes, and nerve cells. In one embodiment, the dermis and/or epidermis comprises a decellularized nipple, a decellularized areola, or a decellularized nipple attached to a decellularized areola. In some embodiments, the collagen comprises a Type I collagen, a Type III collagen, a Type IV collagen, a Type VI collagen, or a combination thereof. In some embodiments, the composition further comprises further a cross-linking agent. In embodiments, the cross-linking agent comprises glutaraldehyde, genipin, or a combination thereof. In some embodiments, the composition comprises a decellularized human nipple. In some embodiments, the composition is of a decellularized human areola. In some embodiments, the composition is of a decellularized human nipple attached to a decellularized human areola. In some embodiments, the composition substantially retains laminin, fibronectin, and/or collagen. In some embodiments, the composition has been at least partially repopulated by cells after decellularization. In some embodiments, the cells repopulating the composition are melanocytes. In some embodiments, the melanocytes are derived from cells from the subject. In some embodiments, the cells repopulating the composition are keratinocytes. In some embodiments, the keratinocytes are derived from cells from the subject. In some embodiments, the cells repopulating the composition are nerve cells. In some embodiments, the nerve cells are derived from cells from the subject. In some embodiments, the decellularized dermis and/or decellularized epidermis can be incubated with cells exogenous to the decellularized dermis and/or decellularized epidermis under conditions conducive to repopulating the decellularized dermis and/or decellularized epidermis with the exogenous cells or cells derived from the exogenous cells.

An aspect of the invention is directed to implantable (for example, implantable on the surface of a subject) surgical grafts comprising the composition of any one of claims 1 to 6. The implantable graft is suitable for grafting to a subject. The graft comprises a composition consisting essentially of decellularized dermis and/or decellularized epidermis (e.g., comprising a decellularized nipple, a decellularized areola, or a decellularized nipple attached to a decellularized areola), wherein the decellularized dermis and/or decellularized epidermis retains at least one matrix molecule selected from the group consisting of laminin, elastin, fibronectin, and/or collagen; wherein the composition further comprises exogenous seeded cells comprising a combination of keratinocytes, melanocytes, and nerve cells. In some embodiments, the surgical graft comprises a decellularized human nipple. In some embodiments, the surgical graft is of a decellularized human areola. In some embodiments, the surgical graft is of a decellularized human nipple attached to a decellularized human areola. In some embodiments, the surgical graft substantially retains laminin, fibronectin, and collagen. In some embodiments, the surgical graft has been at least partially repopulated by cells after decellularization. In some embodiments, the cells repopulating the surgical graft are melanocytes. In some embodiments, the melanocytes are derived from cells from the subject. In some embodiments, the cells repopulating the surgical graft are keratinocytes. In some embodiments, the keratinocytes are derived from cells from the subject. In some embodiments, the cells repopulating the surgical graft are nerve cells. In some embodiments, the nerve cells are derived from cells from the subject. In some embodiments, the decellularized dermis and/or decellularized epidermis can be incubated with cells exogenous to the decellularized dermis and/or decellularized epidermis under conditions conducive to repopulating the decellularized dermis and/or decellularized epidermis with the exogenous cells or cells derived from the exogenous cells. In some embodiments, the graft further comprises a cross-linking agent. In some embodiments, the cross-linking agent comprises glutaraldehyde, genipin, or a combination thereof. In further embodiments, the collagen comprises a Type I collagen, a Type III collagen, a Type IV collagen, a Type VI collagen, or a combination thereof.

An aspect of the invention is directed to a decellularized nipple, areola, or nipple attached to an areola for use in grafting to the chest of a subject with Poland Syndrome, wherein at least one of the decellularized nipple, areola, or nipple attached to the areola when grafted onto the chest of the subject with Poland Syndrome promotes regeneration and/or growth of at least one of the decellularized nipple, areola, or nipple attached to the areola, which is/are grafted on the chest of a subject afflicted with Poland Syndrome, and further wherein the decellularized nipple, areola, or nipple attached to the areola is produced by obtaining a donor nipple, donor areola, or donor nipple attached to an areola; and decellularizing the nipple, areola, or nipple attached to the areola to remove substantially or all cells, wherein at least one matrix molecule is retained in the nipple, areola, or nipple attached to the areola, which matrix molecule is selected from the group consisting of laminin, elastin, fibronectin, and collagen; and further wherein exogenous cells are further seeded onto the nipple, areola, or nipple attached to the areola, and wherein said exogenous seeded cells comprise a combination of keratinocytes, melanocytes, and nerve cells. In some embodiments, the use comprises grafting the decellularized nipple, areola, or nipple attached to the areola onto a chest of the subject. In some embodiments, the decellularized nipple, areola, or nipple attached to the areola is produced by a method further comprising the addition of a cross-linking agent to the decellularized dermis and/or decellularized epidermis (comprising a decellularized nipple, a decellularized areola, or a decellularized nipple attached to a decellularized areola) as described herein. In some embodiments, the cross-linking agent comprises glutaraldehyde, genipin, or a combination thereof. In some embodiments, the seeded nerve cell comprises seeded neurospheres or seeded neuronal cells. In other embodiments, the collagen comprises a Type I collagen, a Type III collagen, a Type IV collagen, a Type VI collagen, or a combination thereof.

Also described herein is the decellularized nipple, areola, or nipple attached to an areola of the invention for use in methods for regenerating a nipple and/or an areola on the chest of a subject afflicted with Poland Syndrome so as to promote regeneration of the nipple, areola, or nipple attached to the areola on the chest of a subject afflicted with Poland Syndrome.

Also described herein is the decellularized nipple, areola, or nipple attached to an areola of the invention for use in methods of promoting the growth of a nipple and/or an areola on the chest of a subject afflicted with Poland Syndrome so as to promote regeneration of the nipple, areola, or nipple attached to the areola on the chest of a subject afflicted with Poland Syndrome.

Other objects and advantages of this invention will become readily apparent from the ensuing description.

### BRIEF DESCRIPTION OF THE FIGURES

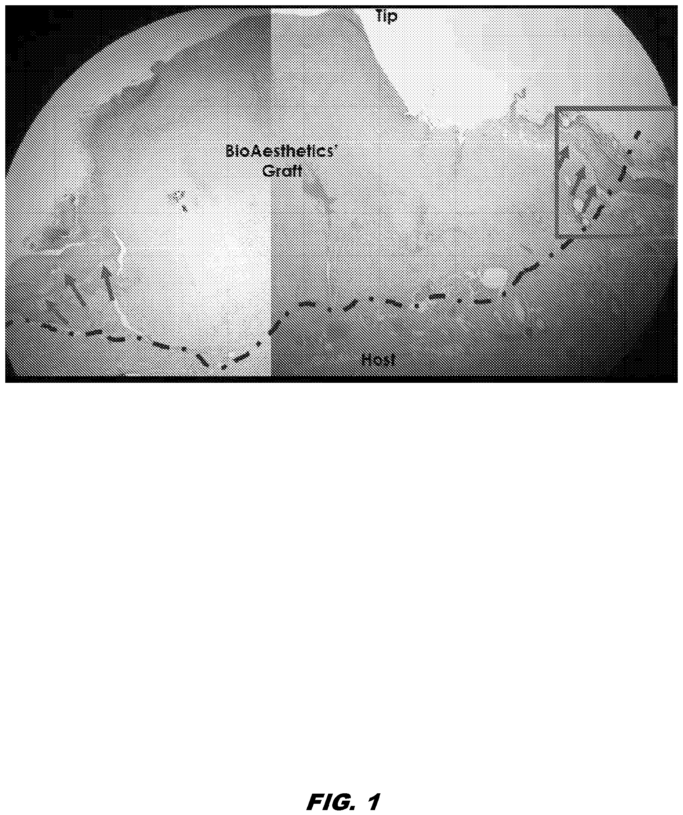

**FIG. 1** is a photomicrograph of a tissue cross section of an H&E stained graft sample of excised nipple with topical sealant at a 1 week time point. The green arrows indicate epidermis with keratin layer forming over the graft.

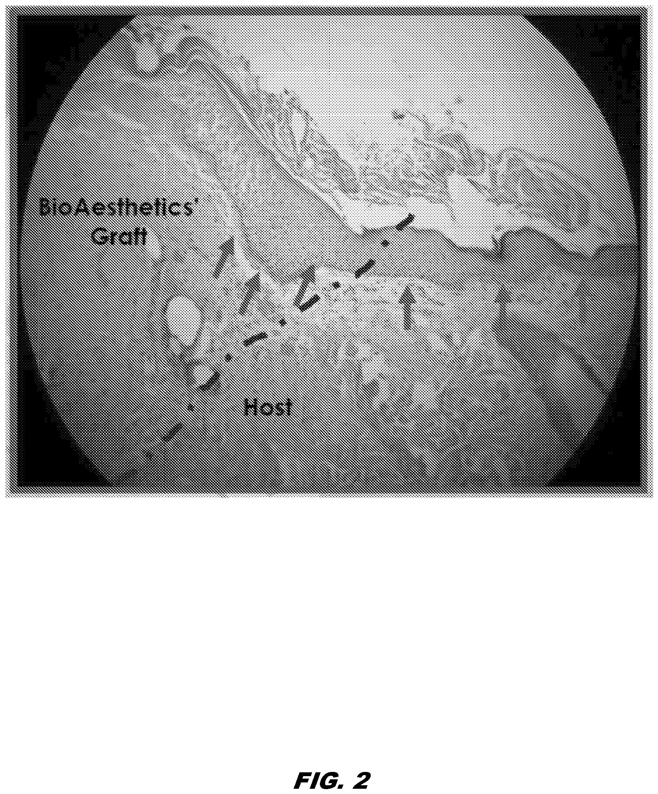

**FIG. 2** is a photomicrograph of a tissue cross section of an H&E stained graft sample of excised nipple with topical sealant at a 1 week time point. The green arrows indicate epidermis with keratin layer forming over the graft. The red arrows indicate NHP host native epidermal layer.

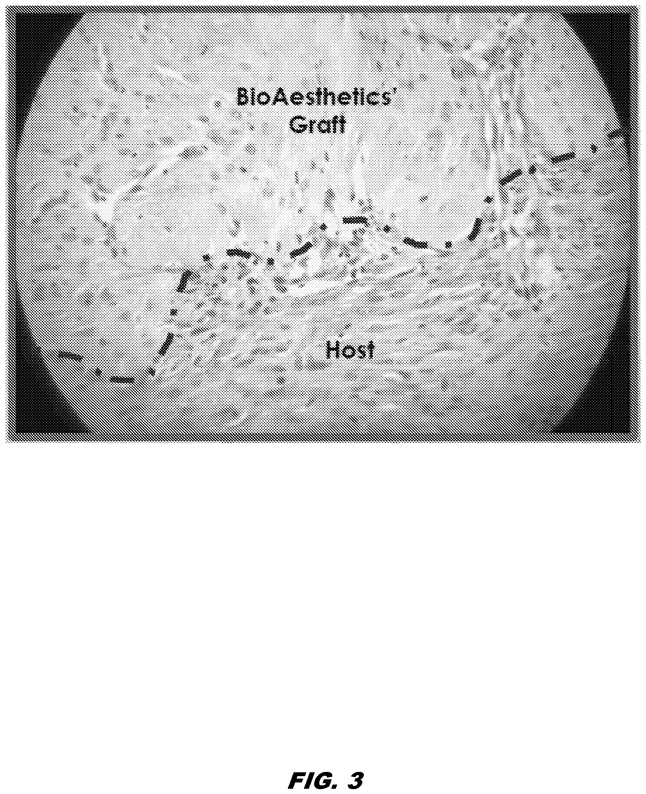

**FIG. 3** is a photomicrograph of a tissue cross section of an H&E stained graft sample of excised nipple with topical sealant at a 1 week time point. The image shows the center of the graft:host dermis contact where host cells are migrating from the host tissue into the graft.

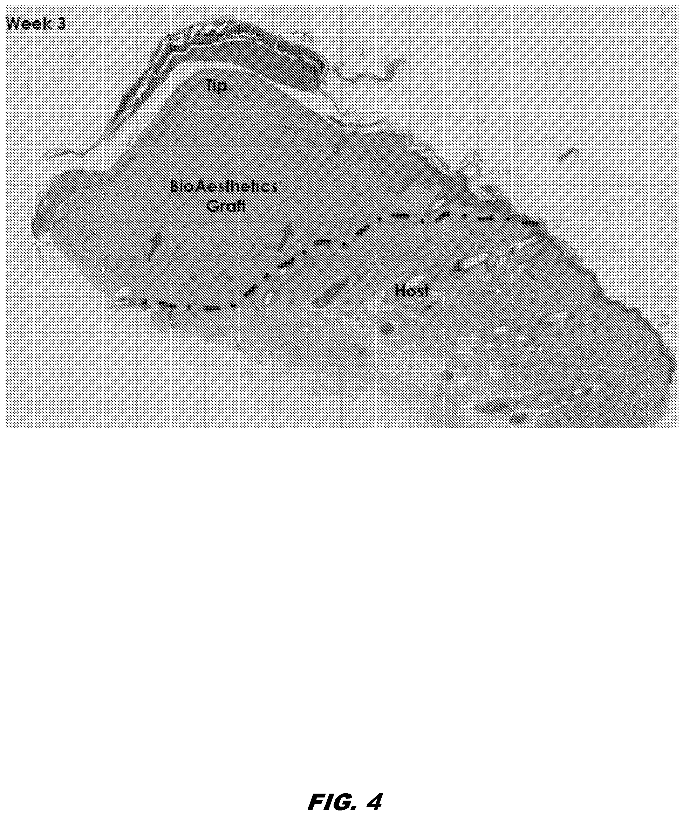

**FIG. 4** is a photomicrograph of a tissue cross section of an H&E stained graft sample of excised nipple with topical sealant at a 3-week time point. The green arrows point to blood vessel-like structures forming in the graft.

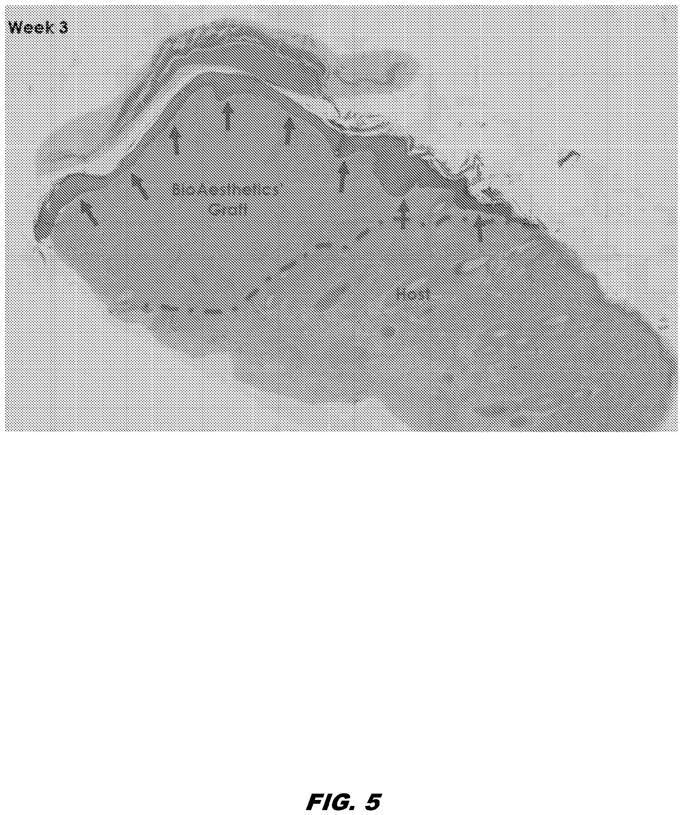

**FIG. 5** is a photomicrograph of a tissue cross section of an H&E stained graft sample of excised nipple with topical sealant at a 3-week time point. The red arrows point to the epidermis with a keratin layer forming over the graft.

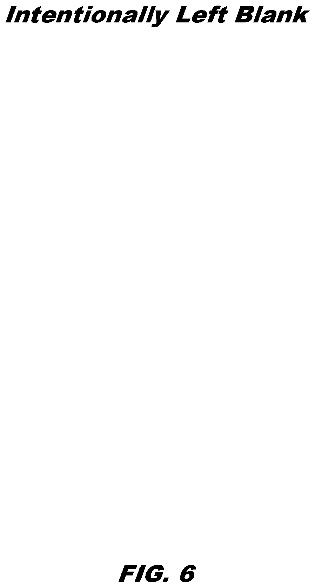

**FIG. 6** is intentionally left blank.
**FIG. 7** shows NAC reconstruction.
**FIG. 8** is a schematic for the decellularization and engraftment process of the NAC for human use. Steps: 1) Removal of a cadaveric NAC for graft generation or removal of patient NAC as part of mastectomy. 2) Decellularization of donor NAC. 3) Acellular NAC graft onlay engrafted onto patient 4) Natural repopulation of acellular NAC scaffold with patient's cells, resulting in regeneration of NAC.
**FIG. 9** shows histology of representative dcl-NHP NAC. (Far left) H&E micrograph of a randomly selected dcl-NHP NAC. Regions in yellow boxes are shown in greater magnification in micrographs 1-3.
**FIG. 10** shows weight and neovascularization (murine). For all results One-way ANOVA with Tukey's posthoc test was performed. * = p<0.05; ** = p<0.01; *** = p<0.001.
**FIG. 11** shows histology of representative dcl-NHP NAC (murine model). (below) H&E micrograph of a randomly selected dcl-NHP NAC. Region in green box are shown in greater magnification in micrographs 1-3.
**FIG. 12** shows body weights of NHP. A) Body weights of NHP. B) Graph of averaged weights from periods of study.
**FIG. 13** shows erythrocytes. A) Graph of erythrocyte cell counts. B) Graph of averaged erythrocyte cell counts from periods of study.
**FIG. 14** shows platelet counts. A) Graph of platelet counts from before, during, and after the engraftment experiment. B) Graph of averaged platelet counts from periods of study.
**FIG. 15** shows erythrocyte properties. A) erythrocyte properties B)Graph of averaged erythrocyteproperty values from periods oHgb(hemoglobin), Hct(hematocrit), Mcv(mean corpuscular volume), Mch(mean corpuscular hemoglobin), Mchc(mean corpuscular hemoglobin per cell), and Rdw(red blood cell distribution width).
**FIG. 16** shows leukocyte profile from NHP peripheral blood. A) Graph of white blood cells from before, during, and after the engraftment experimentB) Graph of averaged leukocyte cell counts from periods of study. WBC(white blood cell), Neu(neutrophil), Lym(lymphocyte), Mon (monocyte), Eos(eosinophil), and Bas(basophil).
**FIG. 17** shows electrolytes. A) Graph of electrolytes from before, during, and after the engraftment experiment. B) Graph of averaged electrolyte levels from periods of study. For all results One-way ANOVA with Tukey's posthoc test was performed. * = p<0.05; ** = p<0.01; *** = p<0.001.
**FIG. 18** shows histology of representative dcl-NHP NAC (below)A) decellularized NHP NAC B) Native vs Decell, C) engraftment orientation, D) Week 1 vs week 6 H&E. E) magnification of center of week 6 from part D (green square).
**FIG. 19** shows Biocompatibility of dcl-NHP-NAC in murine model. **A**) Percent change of weight over time for different grafts. **B**) CD45+ cells (% of total) from blood. C) Number of CD31+ (PECAM-1) cells from histology sections taken for different grafts. N=5 animals per group per time point. **D-G**) Micrographs from H&E stained sections of dcl-NHP-NAC grafts harvested at 21 days post engraftment. Graft is dcl-NHP-NAC. Host is mouse. **E,G**) Magnified regions from B and D, respectively, showing neovasculature (yellow arrows) and re-epithelialization (blue arrows)

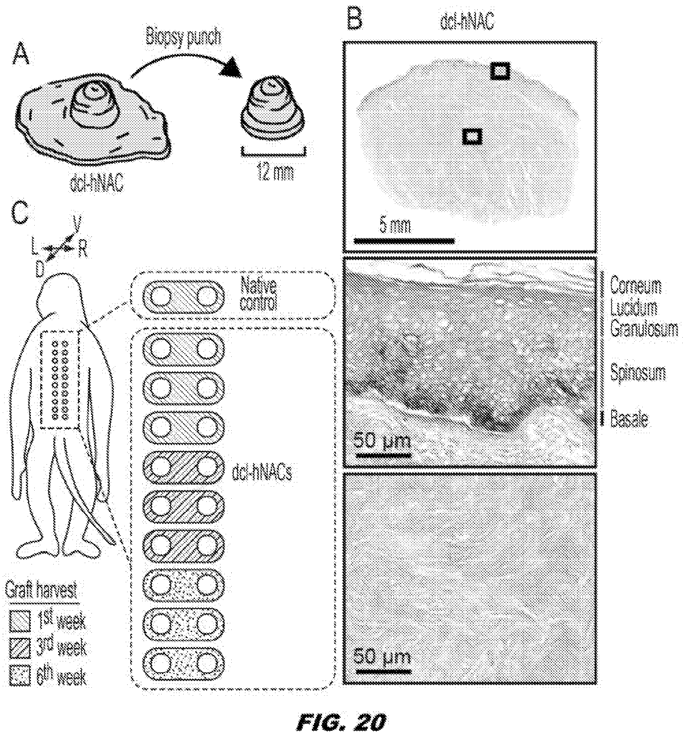

**FIG. 20** shows experimental design for engraftment of dclhNAC grafts on rhesus macaques. **20A**) Decellularized hNAC grafts were biopsy punched (12 mm diameter) prior to engraftment. **20B**) H&E stained section of a punched dclhNAC. Black boxes are magnified regions shown in the middle and bottom panels. The middle panel shows magnification of the epidermis and its strata. The brown pigment is endogenous melanin. The bottom panel shows the center of the graft. Note complete absence of nuclei. **20C**) 18 dcl-hNACs were engrafted along the dorsal midline of rhesus macaques. Grafts were harvested at 1, 3, and 6 weeks post engraftment time points. Native control is the monkey's own NACs which served as surgical controls.

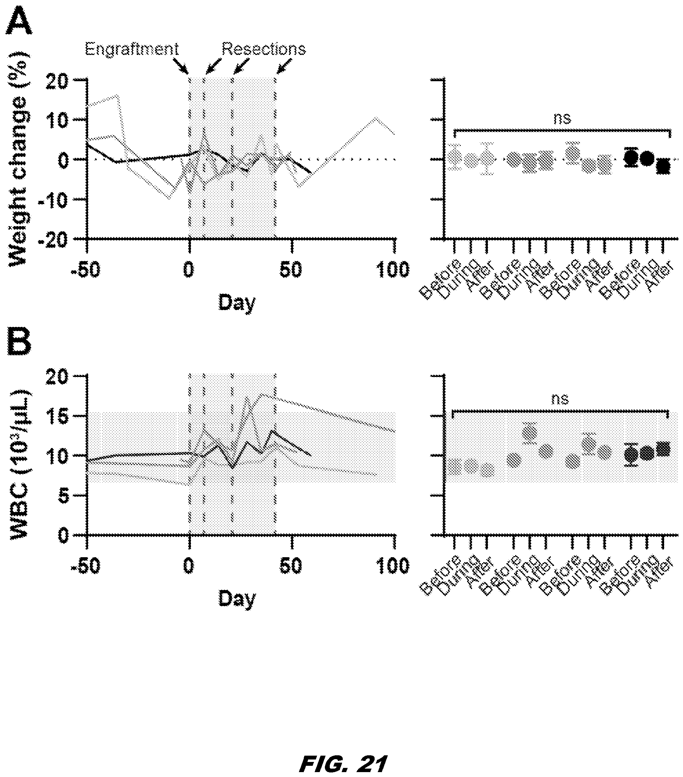

**FIG. 21** are graphs that show the biocompatibility of NHPs engrafted with dcl-NACs. **A**) Percent weight change for all 4 NHPs. Graph to right shows weight change mean±SEM values for periods before, during, and after the study for each NHP. **B**) WBC count for all NHPs. Pink region denotes the normal range. Graph to right shows WBC mean±SEM values for periods before, during, and after the study for each NHP. The grey regions in the left graphs denote the 6 week period during which NHPs had grafts. The dashed vertical lines denote week 0, 1, 3, and 6. Grafts were harvested at 1, 3, and 6 weeks post engraftment, as indicated. One-way ANOVA with Tukey's multiple comparison performed for each experimental period for each NHP. ns = not significant

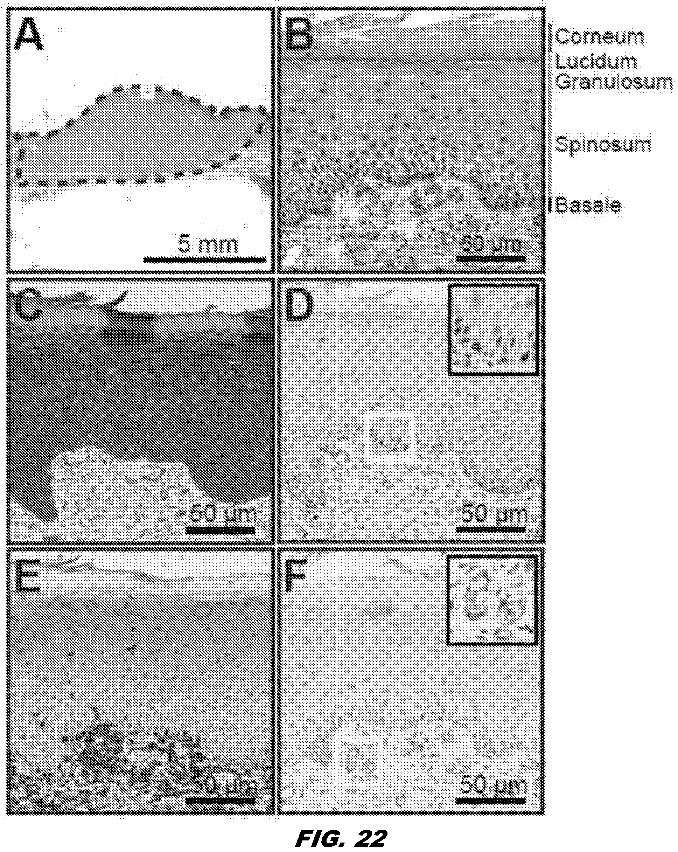

**FIG. 22** are photomicrographs that show histology of dcl-hNAC from NHP 6 weeks post-engraftment. **A**) H&E stained dcl-hNAC graft harvested at week 6 post-engraftment. Graft outlined by blue dotted line. Yellow box magnified in B-F. **B**) Magnified region at apex of NAC graft showing epidermal strata and underlying dermis. Yellow arrows mark blood vessels. **C-F**) IHC staining of same region in B). **C**) Keratinized epidermis (anti-keratin). **D**) Proliferating cells (anti-Ki-67). Inset shows magnified region in yellow box. **E**) Dermal fibroblasts (anti-vimentin). **F**) Neovasculature (anti-PECAM1). Inset shows magnified region in yellow box.
**FIG. 23** are graphs showing the re-epithelialization and neovascularization of dcl-hNAC from NHP study. Left graph shows mean±SEM percent epithelial coverage on dcl-hNAC grafts over time. N=6 grafts/week. Right graph shows the sum blood vessel lumen area±SEM in dcl-hNAC grafts from weeks post engraftment. Quantification performed by measuring area of PECAM+ vessel lumens in 4 randomly sampled regions within grafts. N=6 grafts/week. One-way ANOVA with Tukey's multiple comparison performed for both. ns = not significant, **p<0.01, *** p<0.001.

### DETAILED DESCRIPTION OF THE INVENTION

### Abbreviations and Definitions

Detailed descriptions of one or more preferred embodiments are provided herein. It is to be understood, however, that the present invention may be embodied in various forms. Therefore, specific details disclosed herein are not to be interpreted as limiting, but rather as a basis for the claims and as a representative basis for teaching one skilled in the art to employ the present invention in any appropriate manner.

The singular forms "a", "an" and "the" include plural reference unless the context clearly dictates otherwise. The use of the word "a" or "an" when used in conjunction with the term "comprising" in the claims and/or the specification may mean "one," but it is also consistent with the meaning of "one or more," "at least one," and "one or more than one."

Wherever any of the phrases "for example," "such as," "including" and the like are used herein, the phrase "and without limitation" is understood to follow unless explicitly stated otherwise. Similarly "an example," "exemplary" and the like are understood to be nonlimiting.

The term "substantially" allows for deviations from the descriptor that do not negatively impact the intended purpose. Descriptive terms are understood to be modified by the term "substantially" even if the word "substantially" is not explicitly recited.

The terms "comprising" and "including" and "having" and "involving" (and similarly "comprises", "includes," "has," and "involves") and the like are used interchangeably and have the same meaning. Specifically, each of the terms is defined consistent with the common United States patent law definition of "comprising" and is therefore interpreted to be an open term meaning "at least the following," and is also interpreted not to exclude additional features, limitations, aspects, etc. Thus, for example, "a process involving steps a, b, and c" means that the process includes at least steps a, b and c. Wherever the terms "a" or "an" are used, "one or more" is understood, unless such interpretation is nonsensical in context.

As used herein the term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent up or down (higher or lower).

### Poland Syndrome Etiology

Poland Syndrome is an extremely rare congenital disorder that was first described in 1841 by its principal investigator (PI) Sir Alfred Poland (4) (2). Though conditions can vary greatly between patients, Poland Syndrome is known to present with the complete absence or underdevelopment of the chest muscle (pectoralis major hypoplasia) and webbing of the fingers (syndactyly) (6). In most cases the affected tissue is reserved to one side of the body but in rare instances the disorder can span the entirety of the upper chest and associated limbs. In addition to the primary characteristics of Poland Syndrome the disease may result in underdevelopment of the upper rib cage, shoulder blade and nipple-areolar complexes (2). Individuals who are severely affected present noticeable symptoms at birth, however those with more mild cases may not present until reaching adolescent stages (5). In order for a patient to be diagnosed with Poland Syndrome, they have an abnormality of the pectoralis major in addition to one of the less common areas, such as the nipple-areolar complex (NAC).

Subjects afflicted with Poland Syndrome are typically missing part of the pectoralis major (e.g., missing a large section of the muscle that runs from the upper arm to the sternum). Breast and nipple abnormalities can also occur in subjects with Poland Syndrome. Some subjects with Poland Syndrome have only absence of the breast tissue, while others may be missing all or part of the chest muscle and underlying ribs. For example, some subjects may display aplasia or hypoplasia of the breasts. Some subjects may display aplasia or hypoplasia of the nipples. Some subjects may display aplasia or hypoplasia of the NAC.

### Incidence

There hasn't been any concrete data to suggest exactly what leads to Poland Syndrome and the anomaly is known to be sporadic (10). Though rare in nature Poland Syndrome is widely believed to be under reported and diagnosed (4). A lack of documentation has led to an inability to confirm the exact statistics behind the prevalence of the disorder. The National Organization for Rare Disorders reports incidences ranging from approximately 1 in 10,000 to 1 in 100,000 (5). The U.S. National Library of Medicine estimates that as many as 1 in 20,000 infants are affected (6). While the disease is rare across genders, males are affected three times as often as women are (10). In addition, the right side of the body is affected twice as often as the left side in reported cases (10). A clinical study describing 113 affected patients reported that of their participants 57.5% had breasts and nipples in superior localization (1). Hypothelia (reduced nipple and areola size) and athelia (complete absence of nipple and areola) was observed in 61% and 2.6% of patients, respectively (1).

### Nipple-Areola Complex

The nipple-areolar complex develops over the 12-16 week period of gestation with the differentiation of mesenchymal cells (11). As the breasts mature with puberty the NAC reach full development at which point they provide various functions. NAC abnormalities are generally present at birth but may become more evident as the breast matures and expands. The incidence of hypothelia and athelia in Poland Syndrome patients, accompanied by a lack of permanent repair options, presents a viable reason to explore more aesthetically successful treatment options. The "areola" refers to the small circular area around the nipple.

### Available Surgical Measures

Surgical measures are recommended for individuals who have completed growth and have no life-threatening complications (1). Various surgical techniques performed alone or in combination can provide reconstructive value to patients affected by Poland Syndrome. Some techniques utilize breast implants, customized chest wall implants and the injection of autologous fat cells (12). An increasingly common procedure utilizes a latissimus dorsi muscle transposition and is indicated when this muscle is spared in patients (1). Latissimus dorsi transposition stabilizes the chest wall and provides a symmetrical aesthetic as compared to the patient's developed side but it is important to note that surgery should be withheld until complete development has occurred to preserve symmetry (1). While there has been success in reconstructing underdeveloped pectoralis tissue in affected individuals, the same cannot be said for the nipple-areolar complex.

### Epidermal/Dermal Compositions and Grafts Comprising Same

The invention is directed to compositions consisting essentially of substantially decellularized dermis and/or substantially decellularized epidermis, wherein the dermis and/or epidermis substantially retain(s) at least one matrix molecule that is selected from the group consisting of laminin, elastin, fibronectin, and collagen, and wherein the decellularized dermis and/or decellularized epidermis further comprises exogenous seeded cells comprising a combination of keratinocytes, melanocytes, and nerve cells. A "substantially decellularized" tissue, such as the epidermis and/or dermis (e.g., the nipple and/or areola) described herein can refer to a tissue or structure where about 85%, about 90%, about 91%, about 92%, about 93%, about 94%, about 95%, about 96%, about 97%, about 98%, about 99%, or more of the cells present in the tissue or structure have been removed. In some embodiments, each succeeding higher percentage of cell removal is preferred to the lower percentages. The percentage reduction in the number of cells can be determined by, for example, counting by visual inspection the number of cells visible in samples pre- and post-decellularization, along with DAPI staining to visualize nuclei. "Substantially retaining at least one matrix molecule" can refer to a decellularized structure, for example the dermis or epidermis (such as a decellularized nipple or a decellularized areola, or a decellularized nipple attached to the areola) undergoing immunohistochemical analysis of the extracellular matrix where the presence of the matrix molecule can be seen throughout the ECM and that, for quantifiable proteins, ECM samples show that the sample retains about 30%, about 40%, about 50%, about 60%, about 70%, about 80%, about 90%, about 95%, about 96%, about 97%, about 98%, about 99% or more of the amount of the matrix molecule is present in the extracellular matrix prior to decellularization, with higher percentages being more preferred than lower ones.

A "decellularized" biological tissue or structure (such as the dermis or epidermis herein), for example, can refer to removing most or all of the cells of the tissue or structure while the extracellular matrix (ECM) is substantially preserved in addition to cell adhesion molecules. The extracellular matrix is a complex network of macromolecules filling the extracellular space in a tissue (such as the dermis and/or epidermis that can comprise a nipple and/or areola). The extracellular matrix has three main components: (1) viscous proteoglycans (e.g., glycosaminoglycans (GAGs) covalently linked to proteins ), such as hyaluronan, heparan sulfate, keratan sulfate, chondroitin sulfate, and dermatan sulfate; (2) insoluble collagen fibers (proteins that provide strength) and elastin (proteins that provide resilience); and (3) soluble, fibrous ECM proteins (including fibronectin, and laminin) that bind proteoglycans and collagen fibers to receptors on the cell surface. An "extracellular matrix fibrous protein" and "matrix molecule" each can refer to a fibrous protein of the extracellular matrix, such as fibronectin, laminin, elastin or collagen. In some embodiments, collagen can comprise a Type I collagen, a Type III collagen, a Type IV collagen, a Type VI collagen, or a combination thereof.

For example, a nipple either alone or in combination with the accompanying areola (for example, attached to the areola), can be removed from a subject (e.g., self or non-self), from a cadaver, or from a non-human primate. Such removed tissues/structures can be referred to as a "donor nipple and/or donor areola", and can be decellularized while retaining their natural gross structures, microarchitecture, and matrix molecules, including collagen, fibronectin, elastin and glycosaminoglycans. "Attached" as it relates to a nipple (for example, "attached" to an areola) can refer to a nipple that has been excised from a donor as a unit with the areola surrounding it.

In some embodiments, the decellularized dermis and/or decellularized epidermis compositions can further comprise a cross-linking agent. Crosslinking agents are useful for preventing degeneration of the structural integrity of the scaffold that remains after decellularization, enhancing mechanical strength and reducing calcification of the matrix. For example, the cross-linking agent can bind to proteoglycans, fibronectin, and collagen fibers and other components of the ECM milieu. Non-limiting examples of a cross-linking agent include glutaraldehyde, carbodiimide (1-ethyl-3-(3-dimethyl aminopropyl)-carbodiimide), epoxy compounds, six methylene diisocyanate, glycerin, alginate, genipin, ordihydroguaiaretic acid, proanthocyanidin, tannic acid, collagen, and epigallocatechin gallate.

The decellularized dermis and/or decellularized epidermis compositions further comprise exogenous seeded cells. These decellularized tissues can act as structural scaffolds by which exogenous seeded cells can migrate and readily repopulate. Culturing of these cells is further described herein. In some embodiments, cells from the native tissue (e.g., the host subject) can also migrate into the structural scaffolds created through the decellularization process and readily repopulate the matrix.

For example, the decellularized dermis and/or decellularized epidermis can be seeded and incubated with cells exogenous to the dermis and/or epidermis under conditions conducive to repopulating the decellularized dermis and/or decellularized epidermis with the exogenous cells or cells derived from the exogenous cells. In some embodiments, the exogenous cells can be autologous, homologous (e.g., allogenic), or heterologous. For example, "autologous" refers to biological material (e.g., exogenous cells) that will be introduced into the same individual from whom the material was collected or derived. For example, "homologous" can refer to biological material (e.g., exogenous cells) collected or derived from a compatible donor that will be introduced into a different individual from which the material was collected or derived. For example, "heterologous" can refer to biological material (e.g., exogenous cells) collected or derived from a compatible donor of a different species that will be introduced into an individual. Non-limiting examples of exogenous cells that can be seeded onto (and thus useful for repopulating the decellularized dermis and/or decellularized epidermis) include keratinocytes, melanocytes, nerve cells, stromal cells, stem cells, progenitor cells, bone marrow, and adipose or dermal derived or IPS or differentiated cells from induced pluripotent stem cells (IPSCs). In some embodiments, keratinocytes readily migrate and repopulate decellularized dermis and/or decellularized epidermis. In some embodiments, melanocytes readily migrate and repopulate decellularized dermis and/or decellularized epidermis. In some embodiments, nerve cells readily migrate and repopulate decellularized dermis and/or decellularized epidermis. In further embodiments, the nerve cells can be neurospheres or neuronal cells. For example, "exogenous" relates to cells that have been introduced (e.g., seeded) to recellularize or repopulate a decellularized tissue such that the cells that did not originate in the decellularized tissue. Without being bound by theory, if a nipple from a subject is decellularized and repopulated with keratinocytes, melanocytes, and/or nerve cells originating from a skin punch taken from the same subject, the keratinocytes, melanocytes, and/or nerve cells are still exogenous to the decellularized nipple because they did not originate from the nipple.

To decellularize the dermis and/or epidermis (such as the tissue comprising the nipple, the areola, or the nipple-areola complex), the temperatures of the incubations and the washes can be conducted at about 16°C, about 20°C, about 21°C, about 22°C, about 23°C, about 24°C, about 25°C, or about 26°C. Furthermore, the solutions utilized during the digest incubations can be left unchanged so as to augment cell digestion by not disturbing any endogenous protease activity. The times of the digest incubations can also be doubled, as compared to what is typically practiced in the art. In addition, the concentration of the bile salt used for digests can be at the least doubled, with the concentration of the bile salt raised for example, from about 2% to about 4%, to about 4.5%, to about 5%, to about 5.5%, or to about 6%. Since the dermis/and epidermis tissues can be absent of blood vessels after decellularization (for example, the nipples, areolas, and/or NACs do not have vessels allowing for ready perfusion), the samples can be initially agitated on an orbital shaker set to a rotation speed of about 300 rpm, about 315 rpm, about 320 rpm, about 325 rpm, about 330 rpm, about 335 rpm, or about 340 rpm in order to simulate "perfusion."

For example, an epidermis or dermis sample that is to be decellularized can be contacted with a first detergent and/or surfactant solution for about 48 hours to about 144 hours. In some embodiments, the sample is contacted with a first detergent and/or surfactant solution for about 48 hours, about 72 hours, about 75 hours, about 80 hours, about 85 hours, about 90 hours, about 96 hours, about 100 hours, about 105 hours, about 110 hours, about 115 hours, about 120 hours. As used herein, "about" can be ± 2 hours, which detergent or surfactant can permeate eukaryotic cell membranes and solubilize membrane proteins. Non-limiting examples of detergents useful for decellularization include 4-(1,1,3,3-Tetramethylbutyl)phenyl-polyethylene glycol (Triton^{™} X-100), octylphenoxypolyethoxy-ethanol (IGEPAL^{®} CA-630), CHAPS (3-[(3-Cholamidopropyl)dimethylammonio]-1-propanesulfonate), sodium deoxycholate, sodium dodecyl sulfate, saponin, and polyethylene glycol. After incubation with a detergent or surfactant, the epidermis and/or dermis sample can be washed with water for about 1 hour, about 2 hours, about 3 hours, about 4 hours, about 5 hours, about 6 hours, about 7 hours, about 8 hours, about 10 hours, about 12 hours, or about 16 hours. The epidermis and/or dermis sample can then be contacted with an appropriate soluble bile salt as a second detergent for about 48 hours, about 72 hours, about 75 hours, about 80 hours, about 85 hours, about 90 hours, about 96 hours, about 100 hours, about 105 hours, about 110 hours, about 115 hours, or about 120 hours. Non-limiting examples of sodium salts of bile acids include sodium cholate, sodium deoxycholate, sodium glycocholate, sodium taurocholate, and sodium taurodeoxycholate, which can be purchased from, for example, Sigma-Aldrich Corp. (St. Louis, Mo.). Although these salts have sodium as the cation, other cations can be used to form a salt of a bile acid for use as described herein (e.g., potassium (K+)) provided the resulting bile salt is soluble and can decellularize the dermis and/or epidermis.

In some embodiments, the decellularization of the epidermis and/or the dermis comprises incubating the epidermis and/or dermis sample with at least one detergent at about room temperature for about two days. In some embodiments, the method also comprises washing away the first detergent from the epidermis and/or dermis sample after the first incubation and subsequently incubating the epidermis and/or dermis sample with a second detergent. In some embodiments, the decellularization is of substantially all epidermal cells and substantially all dermal cells in the sample.

The invention described herein further provides for implantable surgical grafts for grafting to a subject according to claim 7. As described herein, the graft can comprise a decellularized epidermis and/or decellularized dermis (such as a tissue sample comprising a decellularized nipple, a decellularized areola, or a decellularized nipple attached to a decellularized areola), which decellularized epidermis and/or decellularized dermis (such as the nipple, areola or nipple attached to an areola) substantially retains at least one matrix molecule selected from the group consisting of laminin, elastin, fibronectin, and collagen. The graft as described herein can also be incubated with exogenous seeded cells, such as a keratinocyte, a melanocyte, a nerve cell, or a combination thereof. The graft as described herein can further comprise a cross-linking agent. A "graft," for example, can refer to a structure or composition that is implanted or attached to a subject in order to replace an anatomical feature or to correct an anatomical defect. The grafts described herein, for example, are useful for replacing epidermal and/or dermal structures (such as nipples, or areolas, or a combination of the two structures), that have abnormally developed or are altogether absent.

For example, a decellularized nipple, a decellularized areola, or a decellularized NAC according to the invention can be for use in a method in which the decellularized nipple, decellularized areola, or decellularized NAC is grafted onto a prepared bed on a subject in need thereof. In these embodiments, cells from the prepared bed (such as keratinocytes, skin stem cells, melanocytes, nerve cells, and fibroblasts) can migrate into the decellularized graft and repopulate it. In some embodiments, the migration of cells into the graft is facilitated by (a) placing the graft on the subject on the prepared bed and (b) coating the graft and the junction where the graft adjoins the subject's skin with a biocompatible substance. For example, the biocompatible substance (or occlusive coating) can be a tissue sealant, a tissue adhesive, tissue glue, or a surgical glue. For example, "biocompatible" refers to a material which is not toxic, not injurious or not inhibitory to mammalian cells, tissues, or organs with which it comes in contact. Furthermore, when the material is in use with respect to a graft does not induce an immunological or inflammatory response sufficient to be deleterious to the subject's health or to engraftment of the graft. Other biocompatible occlusive coatings that provide an air sealing barrier, such as Fibrin glues, can be used. A fibrin sealant, TISSEEL^{®}, is commercially available, as are the sealants BIOGLUE^{®} and DuraSeal^{®}. Non-limiting examples of a tissue sealant include cyanoacrylates (such as high viscosity 2-octyl cyanoacrylate (for example, sold commercially under the names DERMABOND^{®} (Ethicon unit of Johnson & Johnson) and Sure+Close^{®}II)), polyvinylpyrrolidone (water based), ethyl cellulose, pyroxylin/nitrocellulose or poly(methylacrylate-isobutene-monoisopropylmaleate) (alcohol based), latex, and acrylate or siloxane polymers (hexamethyldisiloxane or isooctane solvent based).

In some embodiments, epidermis and/or dermis (for example, comprising a nipple, an areola, or an NAC) can be first decellularized and then repopulated in culture in whole or in part before being grafted onto a subject. In some embodiments, a skin punch taken from a subject can be used as a source of keratinocytes, melanocytes, nerve cells, or a combination thereof, with which to seed the decellularized donor epidermis and/or dermis (for example, comprising a nipple, an areola, or an NAC). Methods of dissociating cells from a skin sample while retaining cell viability are well known in the art and are set forth in standard sources, such as Freshney, R., Culture of Animal Cells: A Manual of Basic Technique and Specialized Applications (John Wiley and Sons, Inc., Hoboken, N.J., 6th Ed. 2010). The skilled artisan can choose the particular dissociation protocol to obtain keratinocytes, melanocytes, nerve cells, or a combination thereof. For example, if a Poland Syndrome subject has a nipple and/or areola which is healthy, one or more skin punches can be taken from the healthy nipple or areola. These punches would then provide a population of melanocytes, nerve cells, as well as keratinocytes, and can provide a more natural color when used to repopulate the NAC as well as sensation in the surrounding NAC area.

Once the cells to be seeded are dissociated, the cells can contact the decellularized epidermis and/or decellularized dermis (for example, comprising a decellularized nipple, a decellularized areola, or a decellularized NAC). However, the dissociated cells can be first expanded in culture, then placed in contact with the decellularized epidermis and/or decellularized dermis (for example, comprising a decellularized nipple, a decellularized areola, or a decellularized NAC). One of skill in the art can seed exogenous cells onto the decellularized epidermis and/or decellularized dermis by placing the decellularized structures into culture medium containing the dissociated, or dissociated and expanded, cells and allowing the cells to migrate into the decellularized epidermis and/or decellularized dermis and repopulate the structures. In some embodiments, cells can be injected into one or more places in the decellularized epidermis and/or decellularized dermis, such as into the interior, in order to accelerate repopulation of the decellularized structures.

The decellularized epidermis and/or decellularized dermis (e.g., comprising a decellularized nipple, a decellularized areola, or a decellularized NAC) are partially repopulated by a combination of keratinocytes, melanocytes, and nerve cells that was seeded before grafting. The combination of keratinocytes, melanocytes, and nerve cells can migrate into the graft from the surrounding skin and continue to populate it after it is grafted into place. Once the decellularized epidermis and/or decellularized dermis (e.g., comprising a decellularized nipple, a decellularized areola, or a decellularized NAC) is grafted onto the subject, it can be covered with a biocompatible occlusive coating (such as those described herein). The skilled artisan can readily obtain a combination of keratinocytes, melanocytes, and nerve cells from one or more skin punches (either from the same subject or from a compatible donor) according to methods and teachings known in the art. The keratinocytes, melanocytes, nerve cells, or a combination thereof, can be placed in a culture medium suitable for maintenance and stability, from which the cells can then permeate into the graft. In some embodiments, these cells can also be injected into the graft at one or more locations. Grafts of the invention can be maintained in a cell culture medium suitable for maintenance and expansion of keratinocytes (human or non-human); cell culture medium suitable for maintenance and expansion of melanocytes (human or non-human); cell culture medium suitable for maintenance and expansion of nerve cells (human or non-human). Cell culture media utilized by the skilled artisan includes, but is not limited to, for example, Minimal Essential Medium (MEM, Sigma, St. Louis, Mo.; for example MEM alpha modification); Dulbecco's Modified Eagles Medium (DMEM, Sigma); Ham's F10 Medium (Sigma, St. Louis, Mo.); HyClone cell culture medium (GE Healthcare, Pittsburgh, PA); RPMI-1640 Medium (Sigma, St. Louis, Mo.); and chemically-defined (CD) media, which are formulated for various cell types, e.g., CD-CHO Medium (Invitrogen, Carlsbad, Calif.) The culture medium used to grow and expand cells of interest can be serum-free and would not require the use of feeder cells. Suitable media specific for keratinocytes are known in the art and include (but are not limited to): Keratinocyte Growth Medium 2 (PromoCell GmbH, Heidelberg, Germany); Stemline^{™} keratinocyte basal medium (Sigma-Aldrich Corp., St. Louis, Mo.); defined, BPE-free medium supplement (K 3136) (Sigma-Aldrich Corp.), and ATCC's Dermal Cell Basal Medium (PCS-200-030) supplemented with Keratinocyte Growth Kit (PCS-200-040). Suitable media specific for melanocytes are known in the art and include (but are not limited to): ATCC's Dermal Cell Basal Medium (PCS-200-030) supplemented with Melanocyte Growth Kit (ATCC PCS-200-041). Suitable media specific for nerve cells are known in the art and include (but are not limited to): DMEM, 10% FBS, supplemented with NGF and L-glutamine.

### Methods of Treatment

The references to the methods of treatment in this description are to be interpreted as references to the compositions, grafts, decellularized nipple, areola, or nipple attached to an areola, for use in those methods.

The term "treating" can refer to partially or completely alleviating, ameliorating, improving, relieving, delaying onset of, inhibiting progression of, reducing severity of, and/or reducing incidence of one or more symptoms, features, or clinical manifestations of a particular disease, disorder, and/or condition. Treatment can be administered to a subject who does not exhibit signs of a disease, disorder, and/or condition (e.g., prior to an identifiable disease, disorder, and/or condition), and/or to a subject who exhibits only early signs of a disease, disorder, and/or condition for the purpose of decreasing the risk of developing pathology associated with the disease, disorder, and/or condition.

The term "subject" or "patient" can refer to any organism to which aspects of the invention can be administered, e.g., for experimental, diagnostic, prophylactic, and/or therapeutic purposes. Typical subjects to which compositions of the present disclosure may be administered will be mammals, particularly primates, especially humans. For veterinary applications, a wide variety of subjects will be suitable, e.g., livestock such as cattle, sheep, goats, cows, swine, and the like; poultry such as chickens, ducks, geese, turkeys, and the like; and domesticated animals particularly pets such as dogs and cats. For diagnostic or research applications, a wide variety of mammals will be suitable subjects, including rodents (e.g., mice, rats, hamsters), rabbits, primates, and swine such as inbred pigs and the like. The term "living subject" refers to a subject noted above or another organism that is alive. The term "living subject" refers to the entire subject or organism and not just a part excised (e.g., a liver or other organ) from the living subject.

As used herein, "changed as compared to a control" sample or subject is understood as having a level of an analyte or diagnostic or therapeutic indicator (e.g., marker) to be detected at a level that is statistically different than a sample from a normal, untreated, or abnormal state control sample. The diagnostic or therapeutic indicator can be assessment of the growth of the tissue grafted or observation for lack of graft rejection. Determination of statistical significance is within the ability of those skilled in the art, e.g., the number of standard deviations from the mean that constitute a positive or negative result.

In one embodiment, the composition, graft, decellularized nipple, areola, or nipple attached to an areola is for use in a method of treatment of a subject with Poland Spring syndrome. These may by produced by a method comprising first obtaining a donor nipple, donor areola, or donor nipple attached to an areola; then decellularizing the nipple, areola, or nipple attached to the areola to remove all cells, wherein at least one matrix molecule is retained in the nipple, areola, or nipple attached to the areola, that is selected from the group consisting of laminin, elastin, fibronectin, and collagen; and wherein exogenous cells are further seeded onto the nipple, areola, or nipple attached to the areola, wherein the exogenous seeded cells comprise a combination of keratinocytes, melanocytes, and nerve cells. In some embodiments, the method comprises decellularizing a nipple, areola, or nipple attached to the areola to remove all cells, wherein at least one matrix molecule is retained in the nipple, areola, or nipple attached to the areola, that is selected from the group consisting of laminin, elastin, fibronectin, and collagen; and wherein an exogenous cell is further seeded onto the nipple, areola, or nipple attached to the areola, wherein the exogenous seeded cells comprise a combination of keratinocytes, melanocytes, and nerve cells. In other embodiments, the use comprises grafting the decellularized nipple, areola, or nipple attached to the areola onto a chest of the subject. In some embodiments, the method further comprises addition of a cross-linking agent to the decellularized dermis and/or decellularized epidermis (comprising a decellularized nipple, a decellularized areola, or a decellularized nipple attached to a decellularized areola) as described herein. In some embodiments, the cross-linking agent comprises glutaraldehyde, genipin, or a combination thereof. In some embodiments, the seeded nerve cell comprises seeded neurospheres or seeded neuronal cells. In other embodiments, the collagen comprises a Type I collagen, a Type III collagen, a Type IV collagen, a Type VI collagen, or a combination thereof.

The invention also provides for the composition, graft, decellularized nipple, areola, or nipple attached to an areola is for use in methods for regenerating a nipple and/or an areola on the chest of a subject afflicted with Poland Syndrome so as to promote regeneration of the nipple, areola, or nipple attached to the areola on the chest of a subject afflicted with Poland Syndrome. In some embodiments, these may be produced by a method comprising first obtaining a donor nipple, donor areola, or donor nipple attached to an areola; then decellularizing the nipple, areola, or nipple attached to the areola to remove all cells, wherein at least one matrix molecule is retained in the nipple, areola, or nipple attached to the areola, that is selected from the group consisting of laminin, elastin, fibronectin, and collagen; and wherein exogenous cells are further seeded onto the nipple, areola, or nipple attached to the areola, wherein the exogenous seeded cells comprise a combination of keratinocytes, melanocytes, and nerve cells. In some embodiments, the method comprises decellularizing a nipple, areola, or nipple attached to the areola to remove all cells, wherein at least one matrix molecule is retained in the nipple, areola, or nipple attached to the areola, that is selected from the group consisting of laminin, elastin, fibronectin, and collagen; and wherein an exogenous cell is further seeded onto the nipple, areola, or nipple attached to the areola, wherein the exogenous seeded cells comprise a combination of keratinocytes, melanocytes, and nerve cells. In other embodiments, the use comprises grafting the decellularized nipple, areola, or nipple attached to the areola onto a chest of the subject. In some embodiments, the method further comprises addition of a cross-linking agent to the decellularized dermis and/or decellularized epidermis (comprising a decellularized nipple, a decellularized areola, or a decellularized nipple attached to a decellularized areola) as described herein. In some embodiments, the cross-linking agent comprises glutaraldehyde, genipin, or a combination thereof. In some embodiments, the seeded nerve cell comprises seeded neurospheres or seeded neuronal cells. In other embodiments, the collagen comprises a Type I collagen, a Type III collagen, a Type IV collagen, a Type VI collagen, or a combination thereof.

The invention also provides for the composition, graft, decellularized nipple, areola, or nipple attached to an areola is for use in methods of promoting the growth of a nipple and/or an areola on the chest of a subject afflicted with Poland Syndrome so as to promote regeneration of the nipple, areola, or nipple attached to the areola on the chest of a subject afflicted with Poland Syndrome. In some embodiments, these may be produced by a method comprising first obtaining a donor nipple, donor areola, or donor nipple attached to an areola; then decellularizing the nipple, areola, or nipple attached to the areola to remove all cells, wherein at least one matrix molecule is retained in the nipple, areola, or nipple attached to the areola, that is selected from the group consisting of laminin, elastin, fibronectin, and collagen; and wherein exogenous cells are further seeded onto the nipple, areola, or nipple attached to the areola, wherein the exogenous seeded cells comprise a combination of keratinocytes, melanocytes, and nerve cells. In some embodiments, the method comprises decellularizing a nipple, areola, or nipple attached to the areola to remove all cells, wherein at least one matrix molecule is retained in the nipple, areola, or nipple attached to the areola, that is selected from the group consisting of laminin, elastin, fibronectin, and collagen; and wherein exogenous cells are further seeded onto the nipple, areola, or nipple attached to the areola, wherein the exogenous seeded cells comprise a combination of keratinocytes, melanocytes, and nerve cells. In other embodiments, the use comprises grafting the decellularized nipple, areola, or nipple attached to the areola onto a chest of the subject. In some embodiments, the method further comprises addition of a cross-linking agent to the decellularized dermis and/or decellularized epidermis (comprising a decellularized nipple, a decellularized areola, or a decellularized nipple attached to a decellularized areola) as described herein. In some embodiments, the cross-linking agent comprises glutaraldehyde, genipin, or a combination thereof. In some embodiments, the seeded nerve cell comprises seeded neurospheres or seeded neuronal cells. In other embodiments, the collagen comprises a Type I collagen, a Type III collagen, a Type IV collagen, a Type VI collagen, or a combination thereof.

### Kits

The compositions and grafts as described herein can also be provided in a kit. The kit may include (a) a container that contains a composition or a graft as described herein, and optionally (b) informational material. The informational material can be descriptive, instructional, marketing or other material that relates to the methods described herein and/or the use of the composition or the graft for therapeutic benefit. The kit may also include a biocompatible sealant for treating a subject with Poland Syndrome.

The informational material of the kits is not limited in its form. The informational material can include information about production of the composition or the graft, components of the composition or the graft, date of expiration, batch or production site information, and so forth. The informational material may relate to methods of administering or affixing the composition or the graft, e.g., in a suitable form, or mode of administration, to treat a subject with Poland Syndrome. The information can be provided in a variety of formats, include printed text, computer readable material, video recording, or audio recording, or a information that provides a link or address to substantive material. In addition to a composition or graft as described herein, the composition in the kit can include other ingredients, such as a buffer, a stabilizer, or a preservative. The composition or graft can be provided in a sterile form and prepackaged.

The kit can include one or more containers for the composition or grafts described herein. The kit may contain separate containers, dividers or compartments for the composition or graft and informational material. For example, the composition can be contained in a culture plate, and the informational material can be contained in a plastic sleeve or packet. The separate elements of the kit may be contained within a single, undivided container. For example, the composition or graft is contained in a container or culture plate that has attached thereto the informational material in the form of a label. The containers of the kits can be air tight, waterproof (e.g., impermeable to changes in moisture or evaporation), and/or light-tight.

### Citations:

(1) http://www.annalsthoracicsurgery.org/article/S0003-4975(14)02009-8/fulltex
(2) https://www.genome.gov/14514230/
(3) https://www.ncbi.nlm.nih.gov/pmc/articles/PMC5010338/
(4) https://rarediseases.info.nih.gov/diseases/7412/poland-syndrome/cases/25064
(5) https://rarediseases.org/rare-diseases/poland-syndrome/
(6) https://ghr.nlm.nih.gov/condition/poland-syndrome
(7) http://www.amirtaherniamd.com/poland-syndrome/?gclid=Cj0KCQiAuZXQBRDKARIsAMwpUeS9ghjiOjiknz8Wp54u03muTuFQE hU2PuafSltpLoc2aZiN3A-2pR0aAlJGEALw_wcB
(8) https://www.ncbi.nlm.nih.gov/pubmed/28450991
(9) https://www.ncbi.nlm.nih.gov/pubmed/?term=17992143
(10) http://europepmc.org/articles/pmc4410274
(11) Nicholas C. Pashos, Michelle E. Scarritt, Zachary R. Eagle, Jeffery M. Gimble, Abigail Chaffin, Bruce A. Bunnell, "Characterization of an Acellular Nipple-Areolar Complex Reconstruction," Cells Tissues Organs. Jan 2017
(12) https://www.ncbi.nlm.nih.gov/pubmed/18178141

### EXAMPLES

Examples are provided below to facilitate a more complete understanding of the invention. The following examples illustrate the exemplary modes of making and practicing the invention. However, the scope of the invention is not limited to specific embodiments disclosed in these Examples, which are for purposes of illustration only, since alternative methods can be utilized to obtain similar results.

### EXAMPLE 1

Decellularization can be conducted as described in the specification along with introducing a cross-linking agent in the process.

For example, cross-linking agents can be added during the decellularization of dermis and/or epidermis according to protocols practiced in the art (*see*, e.g., J Biomed Mater Res A. 2008 Nov;87(2):308-20. doi: 10.1002/jbm.a.31715. Genipin-induced changes in collagen gels: correlation of mechanical properties to fluorescence. Sundararaghavan HG1, Monteiro GA, Lapin NA, Chabal YJ, Miksan JR, Shreiber DI.; *See also*, Manickam et al., (2014) Current Drug Delivery, 11:139-145).

For example, for use in the decellularization of dermis and/or epidermis as described herein, PBS with a defined concentration of genipin (for example, about 0 mM, about 0.5 mM, about 1 mM, about 2 mM, about 2.5 mM, about 5 mM, about 7.5 mM, or about 10 mM) can be added to the petri dish and the dish placed on a rocker to ensure complete mixing. Collagen gels can be incubated in genipin for a defined period of time (for example, about 1 h, about 2 h, about 4 h, about 6 h, about 8 h, or about 12 h), after which the solution was aspirated, and gels were rinsed generously with PBS.

### EXAMPLE 2

### Acellular Nipple-Areolar Complex Allografts for Use in Breast Reconstruction

Currently strategies for NAC reconstruction are dependent on non-living or non-permanent techniques: tattooing, prosthetics or surgical nipple-like structures. Described herein is a tissue engineering approach that can permit a human NAC onlay-graft during breast reconstruction procedures. By applying decellularization, the removal of cellular components from tissue, to an intact whole donor NAC, the extracellular matrix (ECM) structure of the NAC is preserved; thereby, creating a biologically derived scaffold for cells to repopulate and regenerate the NAC.

Non-human primate (NHP) NAC tissues were used as a model for human tissues. A detergent-based decellularization method was used to derive whole-NAC scaffolds from NHP NAC tissue. In vitro characterization include: cell viability, proliferation/apoptosis, histological analysis, proteomic profile comparative analysis and material analysis. In vivo analysis of biocompatibility, vasculogenesis and feasibility was evaluated using two models, murine and NHP animal models.

In the murine C57BL/6 subcutaneous implant model of biocompatibility and neovascularization, decellularized rhesus-derived NAC grafts were compared to surgery only, native mouse skin, and commercially available porcine-derived acellular dermis, over 3 weeks. To evaluate neovascularization and immune cell infiltrate, peripheral blood, weights, and the implants were collected; flow cytometry and immunohistochemistry was performed. Feasibility was conducted by a non-lethal NHP rhesus macaque onlay-engraftment model, CBC/chem12, and weight was analyzed over the 6 week study, and immunochemistry was performed on each of the 20 NAC engraftments. Statistical significance was determined by two-tailed t-tests or two-way ANOVA with a Tukeyspost-hoc test. In vitro sample size: n>3; in vivo: murine n>5/group; NHP grafts n=6/timepoint

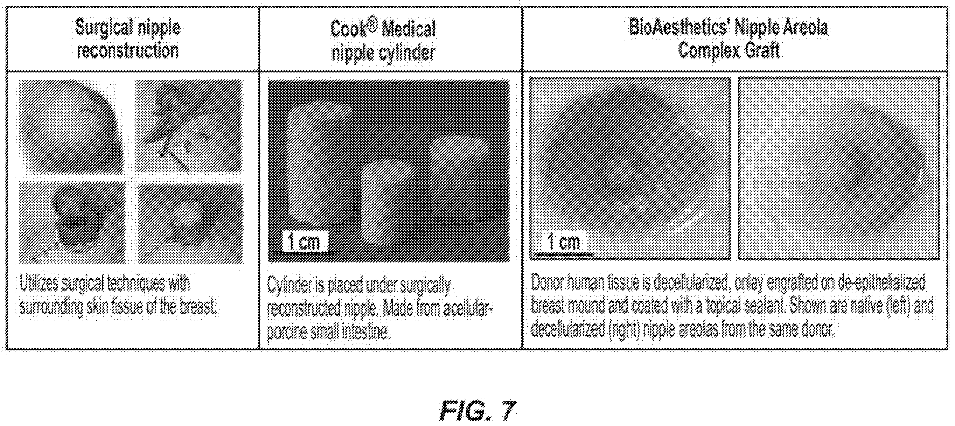

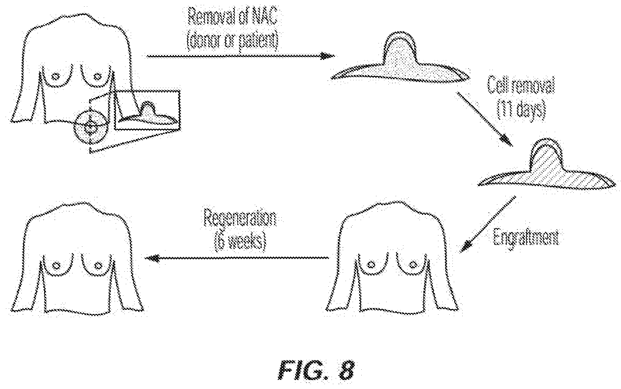

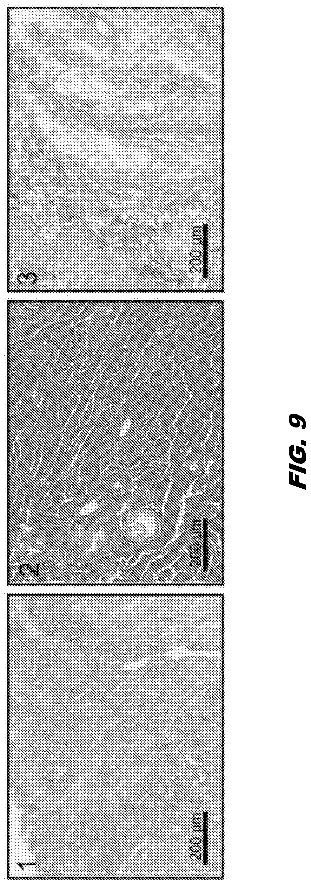

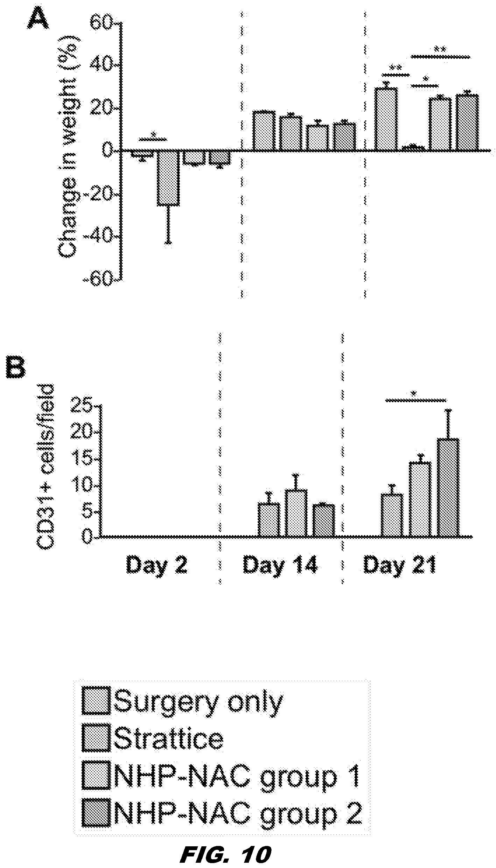

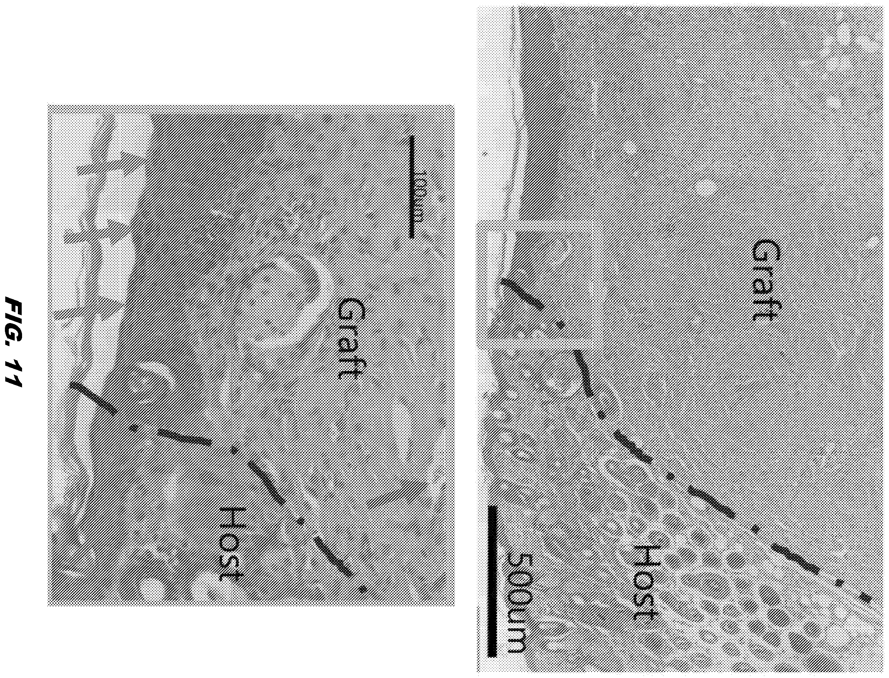

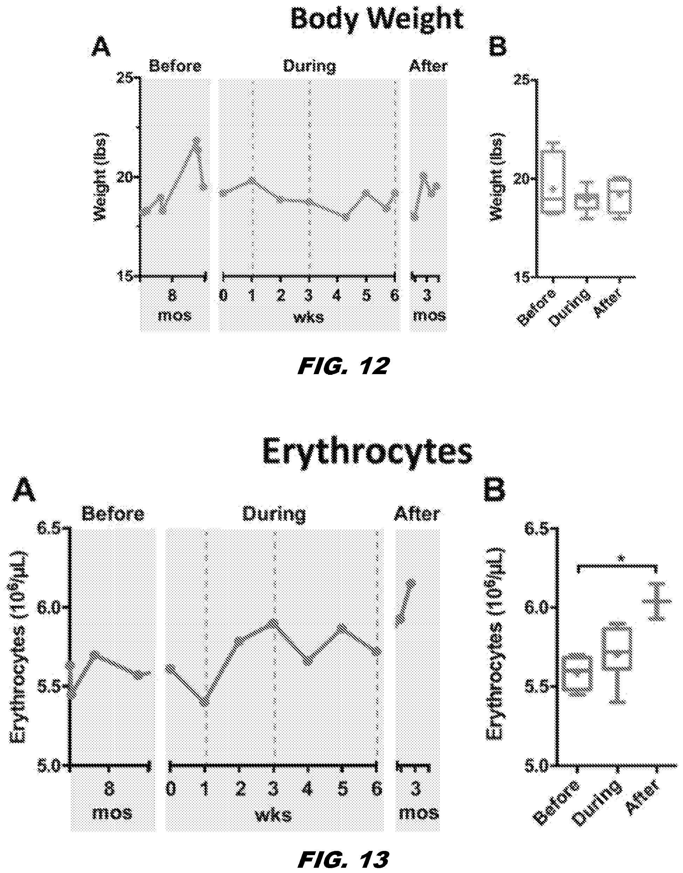

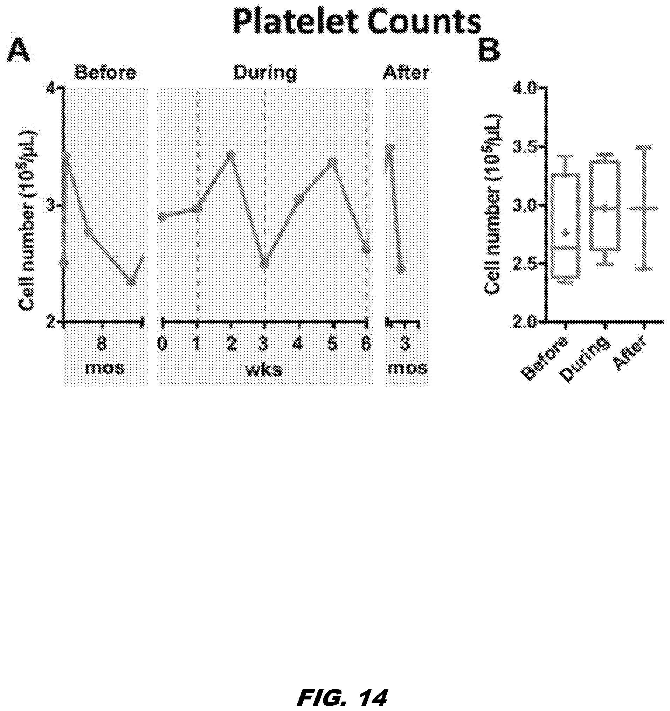

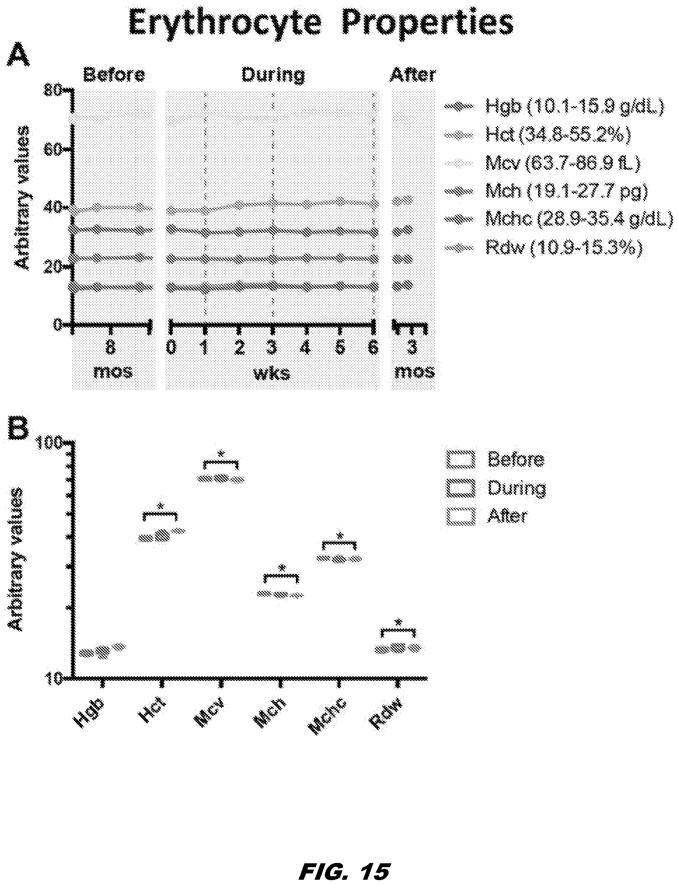

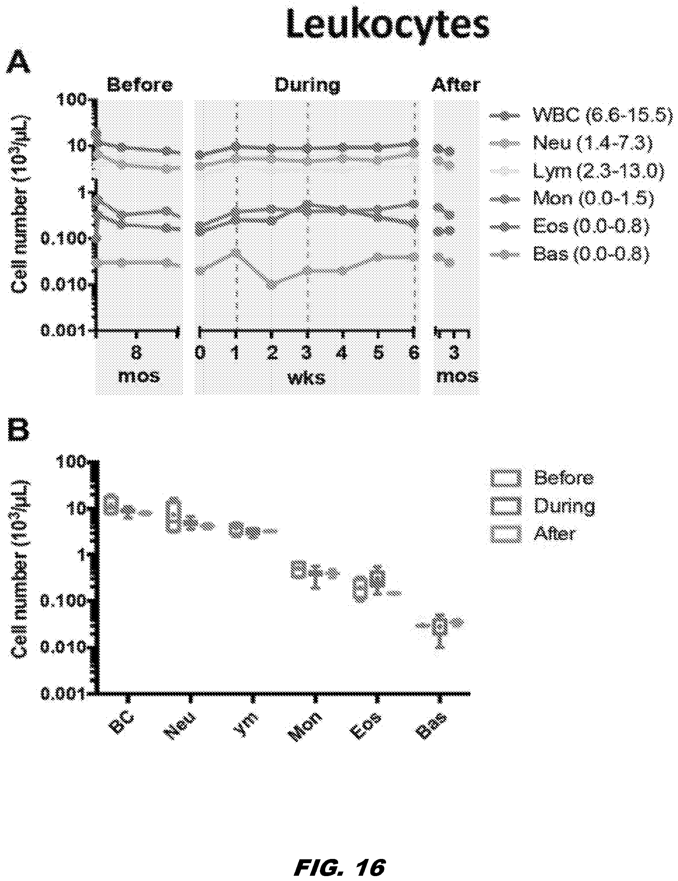

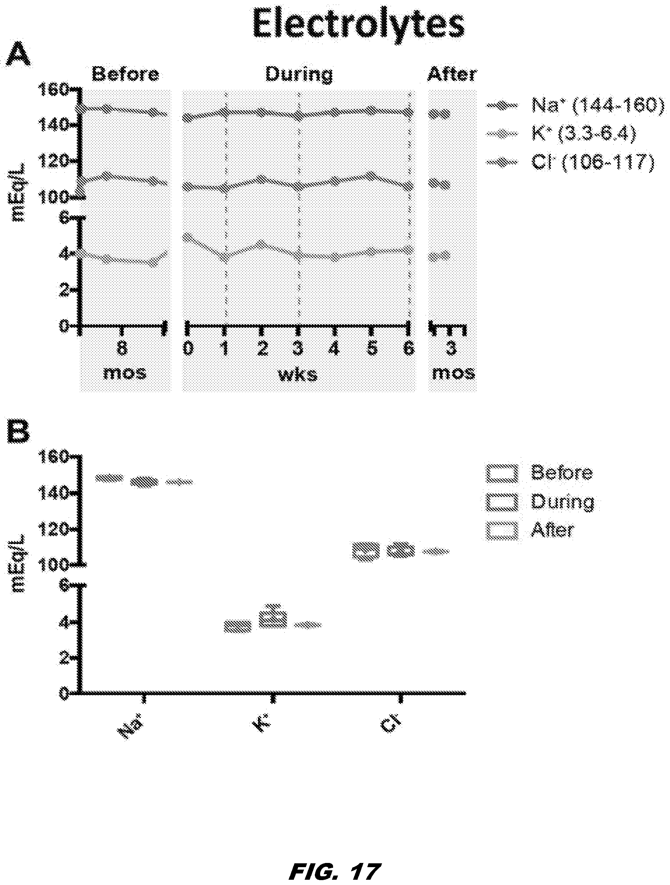

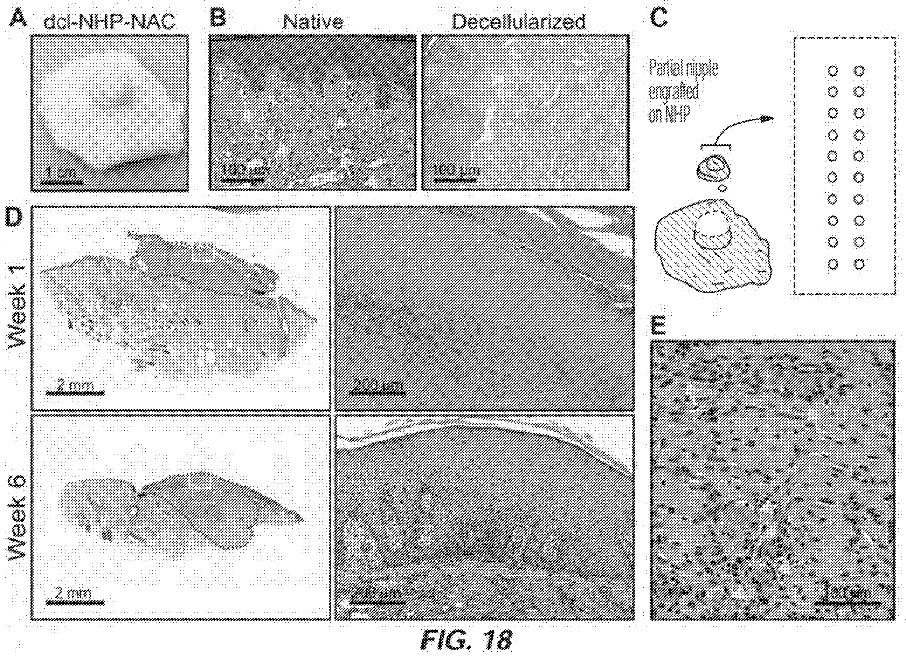

Referring to FIG. 7 to FIG. 18, the data presented here demonstrate that scaffolds are devoid of cells, retain ECM integrity, and a high-degree of bioactivity. The content of collagen and glycosaminoglycans were not significantly altered by the decellularization process; whereas, elastin content was decreased. The proliferation and apoptosis of seeded BMSCs were found to be -65% and <1. 5%, respectively.

*In vivo*: Analysis of flow cytometry data, testing the blood for circulating immune cells, indicated that there was not a significant difference between the decellularized graft and commercially available decellularized dermis control. Analysis of mice weight data shows that those with decellularized NACs had a significantly higher percentage weight gain than did those with commercially available decellularized dermis. CD31+ immunohistochemistry, indicated that neovascularization within the NAC grafts was present at 14 and 21 day time points, significant as compared to control (P<0. 05). No local or systemic immune response was indicated as compared to the control. The feasibility study in the NHP model showed no local or systemic immune response, and re-epithelization starting within 7 days and blood vessel formation within 21 days.

These studies of the decellularized rhesus-derived nipple-areolar complex grafts show significant data that both the decellularization method identified and the biologically-derived whole nipple-areolar complex graft allow for neovascularization and minimum immune response. Together with the electron microscopy imaging of the decellularized NAC scaffold, the enrichment of structural ECM and cytoskeletal proteins demonstrated by proteomics and IHC staining suggest that the microarchitecture is sufficiently intact and the protein landscape has properties favorable for successful epithelialization and vascularization upon implantation. From these in vivo studies, it was observed that the decellularization process and the decellularized implants were able to allow for re-epithelization and blood vessel formation, and maintain a safe biocompatibility profile as compared to the controls. Without wishing to be bound by theory, these data indicate that a whole biologically-derived nipple-areolar complex is a viability and potentially helpful solution in the application of nipple-areolar complex regeneration in subjects afflicted with Poland Syndrome.

### EXAMPLE 3

Current NAC reconstruction options are limited to rubber prostheses, 3D tattoos, and surgical techniques to create NAC-like structures from a patient's own skin tissue or acellular dermal allografts. These approaches produce NACs that either lack physical depth or fail to maintain a protrusion for more than a few months9-11. While commercially available biologically-derived acellular allografts, such as tendon and skin, have been used in reconstructive surgeries for more than 30-years, no such product exists for NAC reconstruction.

One of the biggest challenges to breast reconstruction is the nipple-areolar complex (NAC), a unique tissue made up of a projecting nipple, pigmented skin, keratinized stratified epithelium containing multiple glands, circumferential muscle fibers, rich blood supply, and complex innervation16.

There is no standard, viable method for reliably reconstructing a realistic NAC with enduring nipple projection. Current methods of nipple reconstruction are the limiting factor for a complete breast reconstruction. Current NAC reconstruction options are limited to:
(a) Prostheses: Mass-produced and custom prostheses are NACs constructed from synthetic materials, like polyurethane and/or silicone, that are marketed as matching the shape, size, color, and texture of patients' original nipples26, 27. Prostheses can be attached or removed at will, but secure adhesion is often problematic28. Prostheses are a cheap alternative to surgical procedures26, 27.
(b) Tattoos: Tattoos are the only 2D NAC reconstruction option available. When performed by a professional artist, tattoos can provide the appearance of a 3D nipple and areola but lack physical depth and an actual structure. These tattoos fade within months, requiring ink touchups to color-correct and/or maintain the appearance of projection29, 30. Tattoos are often used with other NAC reconstruction techniques to provide the appearance of pigmentation.
(c) Skin Flap Surgery: A variety of surgical "flap" techniques can construct nipple-like projections. These nipplelike structures may be made from surrounding breast tissue, skin, or cartilage from another part of the body (e.g. back, buttocks, thighs, etc.), or an acellular allograft. Current techniques rely heavily on surgical skill and time. The surgeon must hand-shape local tissues, secondary site tissues, or commercially available acellular dermal sheets. Donor site damage, such as scarring and loss of sensation, frequently occurs when the patient's tissues are used. With current procedures, more than half of the projection is lost within a year30.
(d) Nipple-Sharing Grafts: Although not a common technique, some patients may opt for nipple-sharing grafts. In this surgery, part (up to 50%) of the nipple on the healthy breast is grafted onto the contralateral reconstructed breast31. This option is not feasible for all patients and only available for women who have had a unilateral mastectomy and have a nipple of adequate size31. It is less preferred by some breast reconstructive surgeons over skin flap surgery32.
(e) Cook Medical: Cook Medical's Biodesign^{®} Nipple Reconstruction Cylinder (BD-NRC) is an acellular collagen matrix derived from porcine intestinal submucosa. The BD-NRC is inserted under the skin as a support device during skin flap surgery, thus requiring the same local or secondary site nipple reconstruction. On average, less than 40% of projection is maintained at 1-year post-reconstruction9, 33. These approaches produce NACs that are non-living, non-permanent, lack physical depth, or fail to maintain a protrusion for more than a few months9-11. Because most surgical NAC reconstructions utilize non-homologous soft tissue11, up to 70% loss of nipple projection can occur by the end of the first year post-reconstruction10. In addition, current surgical approaches show variable patient satisfaction outcomes across similar reconstruction techniques - a likely result of differing surgical expertise and skill between surgeons34-37. This can result in patients seeking burdensome revision surgeries when their reconstructed NACs lose projection and alter in general appearance. NAC reconstruction is a significant unmet medical need; patients and physicians need an easy-to-apply, standardized, permanent, and aesthetically pleasing NAC reconstruction option.

***Scientific Premise.*** The inventors have adopted a tissue engineering approach to address the failures of current NAC reconstruction approaches with the primary failure being loss of projection. Without being bound by theory, tissue regeneration can occur when a non-regenerating host's cells repopulate a non-immunogenic scaffold/structure, leading to formation of a living and self-renewing tissue. The search for an appropriate scaffold can lead one to synthesize scaffolds, such as hydrogels and 3-D printed structures, from synthetic or natural polymers. However, native tissue matrices are highly complex, being composed of hundreds of modified extracellular matrix (ECM) proteins that structurally interconnect and possess unique mechanical and signaling properties38-40. This greatly differs from synthesized scaffolds which lack heterogeneity in both polymer nature and structure. Basic observation reveals that skin lays flat or conforms to the shape of a structure whereas the NAC maintains a projection throughout a person's lifetime. The underlying structure of the NAC is significantly different than that of skin. Proteomic analyses show differing compositions and stoichiometries of ECM proteins in human NAC tissue versus neighboring skin with notable differences in collagen types, elastin, and laminin levels. Analyses with histology and cryoSEM demonstrate differing collagen bundle orientations in the skin from the NAC. Collagen bundles in the skin appear more oriented in an orthogonal pattern (e.g., cross-hatch weave) whereas bundles in the NAC have more unidirectional bundles, particularly in the nipple41. These structural differences likely reflect the differing nature of forces these distinct epithelia cope with (i.e. planar stress for skin; compressive force for the nipple).

At present, the body's complex natural ECM architecture cannot be synthetically recreated; however, the body's natural ECM can be used as a scaffold for tissue regeneration through use of donor tissue. To avoid host rejection and increase available tissue for engraftment, tissue engineering has developed methods for removing endogenous cells and DNA from intact donor tissue, resulting in the creation of an acellular scaffold devoid of immunogens. This process, known as decellularization, greatly maintains the native ECM by preserving the insoluble protein and proteoglycan components of the matrix as well as their architectural complexity. Many decellularization methods have been developed that are all very similar in nature42; however, each decellularization method is uniquely tailored to different tissue types (e.g. skin, heart, lung, bone, tendon, NAC, etc). This is largely due to structural differences (i.e. ECM composition, density, stratification, etc.) for each tissue type. While acellular allografts, such as tendon and skin, have been commercially available and used in reconstructive surgeries for more than 30 years, no such product exists for NAC reconstruction. For example, a decellularized human-derived nipple-areolar complex (dcl-hNAC) graft has been created that serves as a scaffold for regeneration of a patient's NAC and that would be engrafted on the exterior of the body on a de-epithelialized wound bed (onlay engraftment) in the anatomical location of the NAC. The use of a dcl-hNAC externally differs from previous uses of acellular allografts, which are primarily indicated for internal use. To derive the product from donor tissue, a decellularization process was rigorously developed, characterized, and published in peer-reviewed journals41,43-45, and meets widely used criteria46.

Current thinking related to organ regeneration/transplantation is to regrow an organ in vitro and transplant the living organ to the patient; however, organs are much more complicated and likely take a longer time to regenerate than the NAC structure, as the NAC does not include glands. In addition, in vitro regeneration followed by transplantation would require blood supply to be established immediately upon transplantation in order for the graft to survive. The benefit of an acellular material is the lack of rejection risk and lack of need for immediate blood supply. Acellular allografts have been utilized in patients for over 30 years and studies have shown that as cells repopulate acellular scaffolds, blood supply follows42. The inventors demonstrated that this also applies for their onlay engrafted dcl-hNACs through biocompatibility and feasibility studies that were performed across different model systems as detailed herein.

From a clinical perspective, reconstructive surgeons are very familiar with acellular allografts since they have been used for decades. Clinical use of the product as described herein is as follows: 1) dcl-hNAC is shipped to surgery center in hydrated form, which allows for off-the-shelf availability, 2) dcl-hNAC enters sterile surgical field, 3) surgeon de-epithelializes breast mound in location of NAC, 4) dcl-hNAC is sutured in location circumferentially and antibiotic ointment or topical sealant (e.g. Dermabond) is applied, 5) NAC area is bandaged with bolster dressing to minimize compression of dcl-hNAC, and finally, 6) wound healing (i.e. re-epithelialization and revascularization of dcl-hNAC by patient cells) occurs in 6-8 weeks.

***NAC Decellularization.*** A decellularization method that can be used accordingly are described in US application publication no. US 2018/0015204 A1 ("Surgical Grafts for Replacing the Nipple and Areola or Damaged Epidermis"). NHP skin is similar to human in its appearance, disease etiology, and approach for clinical care63, 64. Thus, we have analyzed ECM morphology and biocompatibility for both dcl- NHP-NAC and dcl-hNAC scaffolds and found similar results, demonstrating to us that dcl-NHP-NAC scaffolds serve as an informative surrogate/model for dcl-hNAC scaffolds. dcl-NHP-NAC scaffolds were analyzed that underwent the decellularization process (e.g., the process described in US 2018/0015204 A1) for the retention of ECM components such as collagen, glycosaminoglycans (GAGs), and elastin41. Briefly, collagens (types-I and -III) are major ECM components of skin, GAGs are hydrophilic polysaccharides that provide impact retention to the ECM, and elastin fibers are an essential component for skin elasticity. We confirmed the preservation of collagen (Gomori trichrome, H&E) and GAG (Alcian blue) content to levels similar to intact dermis, and detected a decrease in elastin levels (Movat's pentachrome)41. Levels of elastins (soluble tropoelastins, lathyrogenic elastin, α-elastin, and κ-elastin) were measured (Fastin Elastin Assay, BioColor Life Science) in native and decellularized NHP NACs. A reduction in elastin content was observed in dcl-NHP-NACs (7.77±2.84 µg/mg tissue) from native tissue (25.03±2.84 µg/mg tissue) (n=3, p<0.01). A decrease in elastin is highly common in decellularized tissue43, 45. Worth noting, the decreased elastin levels measured are sufficient for the maintenance of native-like mechanical properties43, 45. The relative levels of retained ECM components from the dcl-NHP-NACs were similar to data from other epithelial tissue such as lung from rhesus macaque and rat, and skin from pig43, 45, 65, 66. Additionally, scanning electron cryomicroscopy was performed to visually evaluate extracellular matrix fibers. The integrity of collagen bundles and fibers were maintained both in the dermal and epidermal layers in dcl-NHP-NACs similarly to native NHP-NACs. In summary, dcl-NACs are devoid of immunogenic levels of genomic material, retain overall structures on the micro- and macroscale with ECM fibers remaining intact and structurally similar to native tissue. These data demonstrate that the decellularization process is appropriately optimized and that the extracellular matrix is intact after decellularization.

***Decellularized NACs support cell survival and proliferation in vitro.*** We measured *in vitro* cell viability and apoptosis of rhesus macaque bone-marrow derived stem cells (BMSCs) in the presence of dcl-NHP-NAC scaffolds41. Briefly, dcl-NHP-NAC scaffolds (7x7x2 mm) were seeded with 1 x 10⁶ BMSCs and grown under culture conditions for 7 days, then stained for Proliferating Cellular Nuclear Antigen (PCNA) and with TdT-mediated dUTP Nick-end Labeling (TUNEL) to evaluate proliferation and apoptosis, respectively. Cell proliferation and apoptosis were evaluated after 1, 2, and 7 days (n = 4 grafts/time point). BMSCs survived and proliferated after initial cell seeding through the 7-day end time point. Less than 1% of cells were apoptotic at the time points analyzed and the percentage of cells undergoing proliferation were greater than 60% for all grafts at the time points analyzed. H&E stained tissue sections demonstrated cells attached to the periphery of the acellular scaffold during early time points with cells migrating deeper into the scaffold by day 741. No significant difference was found between the three groups throughout the 48 hours, with cell viability >90% for all. These data indicate the decellularized scaffold provides a non-toxic environment permissible for continuous growth of seeded cells, allowing cells to both adhere and proliferate.

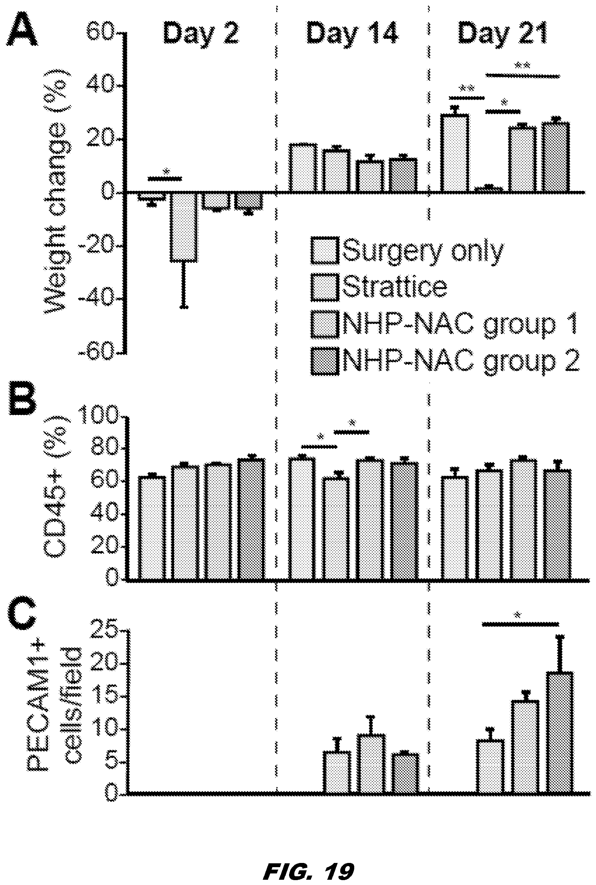

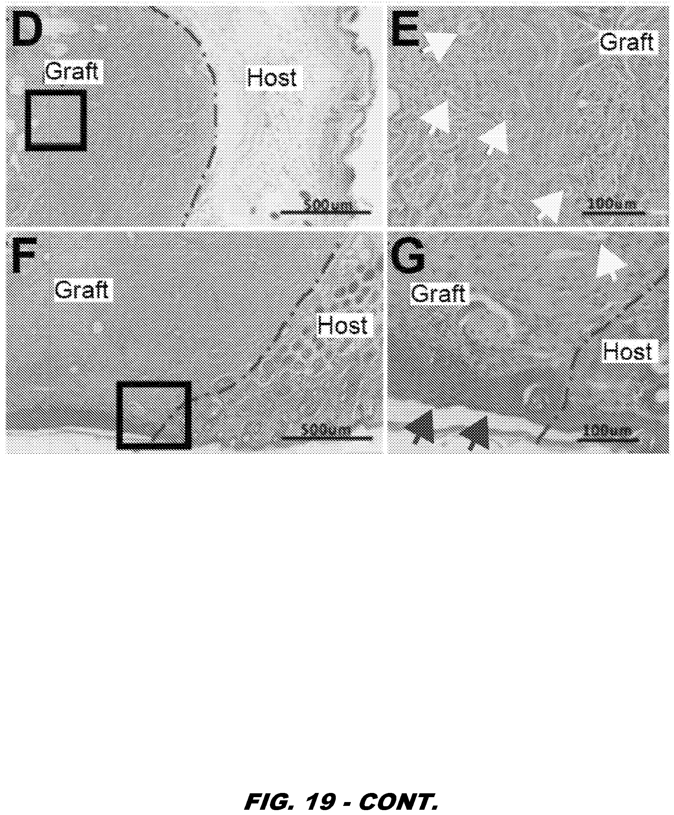

***Decellularized NACs support cell survival and neovascularization in mice.*** Mice (n=120) were subcutaneously implanted with dcl-NHP-NAC grafts and compared to surgery only and Strattice (a widely used, commercially available acellular graft from porcine dermis). Group 2 dcl-NHP-NAC grafts served as a technical duplicate. Grafts and blood samples were collected at 2, 14, and 21 days post-engraftment with weights observed weekly. Changes in mice weights were not different in dcl-NHP-NAC grafts from surgery only controls (**Fig. 19A**). Mice harboring dcl-NHP-NAC grafts showed increased weight gain as compared to Strattice which showed an initial decrease at day 2 and remained unchanged at day 21. We analyzed blood via flow cytometry for CD45+ leukocytes to determine if the dcl-NHP-NAC was immunogenic (**Fig. 19B**). No differences were observed in CD45+ levels in dcl-NHP-NAC grafts as compared to surgery-only control for days 2, 14, and 21. Excised grafts were stained for PECAM- 1 to label endothelial cell-containing vascular lumens. PECAM-1 staining showed neovascularization within the dcl-NHP-NAC graft at the 14- and 21-day time points. Neovascularization levels in the dcl-NHP-NAC occurred at levels similar to or higher than that seen with Strattice (**Fig. 19C**). Blood vessel formation was visible within the dcl-NHP-NACs after 21 days, as seen by staining with H&E (**Fig. 19D-19G**). In some mice with premature suture removal due to animal activity, the scaffolds were exposed to air. These dcl-NHP-NACs exhibited reepithelization (**Fig. 19F, 19G**). These data indicate the dcl-NHP-NACs (a) do not elicit a systemic immune response and (b) encourage neovascularization and re-epithelialization.

***Engrafted decellularized NACs do not elicit adverse systemic responses and are biocompatible in rhesus macaque.*** We are developing the dcl-hNAC to provide surgeons with an off-the-shelf ready product for onlay engraftment onto a patient's breast mound or chest during breast reconstruction. To test both the feasibility of this approach and the biocompatibility of our acellular nipple grafts on a humanlike host, we onlay engrafted multiple decellularized nipple tissue grafts on rhesus macaques (n=4). The experimental design used for a study is shown in **Fig. 20**. Variations in graft number (14 vs 20), graft size (10 mm vs 20 mm), and donor species (hNAC vs NHP-NAC) have been explored in these studies. Briefly, dcl-hNACs were biopsy punched to 12 mm diameter which included the entire nipple and a portion of the areola (**Fig. 20A**). These grafts were completely decellularized yet retained epidermal and dermal substructures, further evidence of the nondestructive decellularization process (**Fig. 20B**). Under sterile conditions, these dcl-hNACs were engrafted along the dorsal midline of an NHP (**Fig. 20C**). On each animal, two surgical controls (native autologous nipples) and 18 dcl-hNAC grafts were onlay engrafted. The dorsal midline region was specifically chosen because tissue flaps from a patient's back (latissimus dorsi flap) are commonly used in breast reconstructions. The dclhNACs were onlay engrafted according to the proposed clinical approach by a plastic surgeon experienced in NAC reconstruction. Briefly, the graft site was de-epithelialized, exposing the dermal bed. The dcl-hNACs were then sutured on top of the dermal bed, covered with ointment-impregnated gauze, and covered with waterproof wound dressings. Commercial NHP jackets were used to prevent manipulation of the bandaged areas. Grafts were resected at 1, 3, and 6 weeks post-engraftment for histological measures of re-epithelialization and neovascularization. The study length of 6 weeks was used as acellular dermal grafts have been previously shown to support neovascularization and detectable blood flow as well as re-epithelialization (human clinical trials) by this timepoint56, 67, 68.

We evaluated systemic responses to grafts by measuring body mass, blood cell counts, and metabolite levels from periods before, during, and after engraftment for all NHPs. There was no abnormal change in weight for any animal during the engraftment period (**Fig. 21A**). Mean percent weight change did not differ from periods before, during, and after engraftment for all animals. Furthermore, white blood cell (WBC) counts showed consistent trends for each animal that remained within the normal range (**Fig. 21B**). Transient increases in WBC counts were found; however, these increases were in response to graft resections that occurred at weeks 1, 3, and 6. To enable histological assessment of grafts, this could not be avoided. Despite this added surgical burden, mean WBC counts did not differ from periods before, during, and after engraftment for each animal. Furthermore, no adverse events were noted during these studies with animals showing normal health and behavior. These data demonstrate that our decellularized nipple grafts are biocompatible and non-immunogenic.

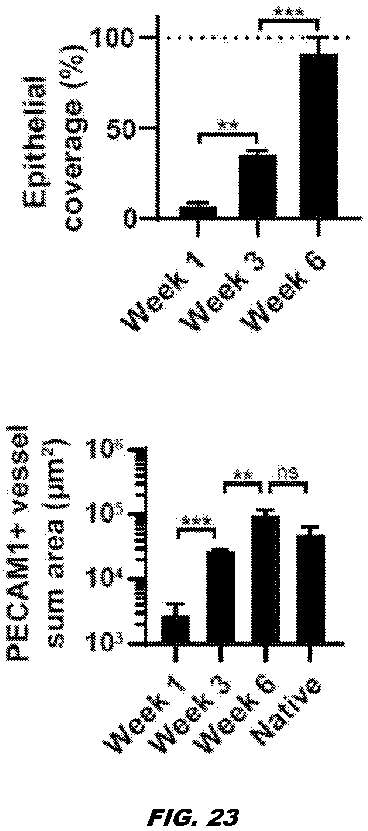

***Decellularized NACs support re-epithelialization and neovascularization on rhesus macaque.*** dcl-hNACs showed robust integration and recellularization on NHPs. Histological analysis of resected grafts showed re-epithelialization is observed within 1 week, with a visible epidermis over the matrix within 3 weeks, and a completely stratified epidermis visible at 6 weeks (**Fig. 22A, 22B**). In the week-6 dcl-hNACs, we detect several hallmarks of normal skin, including a keratin-containing epidermis (**Fig. 22C**), a proliferative basal substratum (**Fig. 22D**), a high density of dermal fibroblasts within the dermis (**Fig. 22E**), and blood vessels (**Fig. 22F**). It is important to remember that these grafts were completely decellularized (see **Fig. 20B**) prior to engraftment and are replete with migrating and dividing cell-types within 6 weeks. Quantification of re-epithelialization and neovascularization of dcl-hNACs showed relatively rapid recellularization within the NHP host (**Fig. 23**). A steady increase in epithelial coverage occurred during the 6 week study period (91.4±8.6% coverage). Neovascular formation occurred within grafts as early as 1 week post engraftment, with more mature vessel formation, as well as increased presence of vessels, occurring over time. Quantification of blood vessel area (measured from PECAM+ luminal stains) within grafts at 6 weeks showed no difference in vessel area from native NHP-NACs (surgical controls, see **Fig. 20C**). These data indicate that our dcl-hNACs support full recellularization and formation of the epidermis, dermis, and vasculature, within the rhesus macaque NHP model.

In our NHP studies, nipple projection was observed at each 6 week endpoint, but not measured. Although this data is critical to demonstrate the superiority of our dcl-hNAC to existing options, limitations of the animal model prevented us from mimicking clinical care expected for a human, specifically related to bandaging. For the NHP studies, multiple bandages were wrapped tightly around the animal's torso to prevent manipulation of the grafts by the animal. This caused significant compression on the grafts. The bandaging in the human clinical setting will include a bolster (i.e. donut) around the dcl-hNAC, thus eliminating bandage-induced compression. Despite the compression bandages in the NHP model, we observed maintenance of nipple projection in a majority of the grafts, although reduced in many. However, the data as a whole demonstrate that the dcl-hNAC is safe and ready for a first-in-human study and ultimately to be used in Poland Syndrome subjects.

## Claims

1. A composition consisting essentially of decellularized dermis and/or decellularized epidermis, wherein the dermis and/or epidermis retain(s) at least one matrix molecule selected from the group consisting of laminin, elastin, fibronectin, and collagen; and wherein the decellularized dermis and/or decellularized epidermis further comprises exogenous seeded cells comprising a combination of keratinocytes, melanocytes, and nerve cells.

2. The composition of claim 1, wherein the exogenous seeded cells originate from a skin punch obtained from the same subject as the decellularized dermis and/or decellularized epidermis, or the exogenous seeded cells originate from a skin punch obtained from a compatible donor.

3. The composition of claim 1 or claim 2, wherein the dermis and/or epidermis comprises a decellularized nipple, a decellularized areola, or a decellularized nipple attached to a decellularized areola.

4. The composition of any preceding claim, wherein the collagen comprises a Type I collagen, a Type III collagen, a Type IV collagen, a Type VI collagen, or a combination thereof.

5. The composition of any preceding claim, further comprising a cross-linking agent.

6. The composition of claim 5, wherein the cross-linking agent comprises glutaraldehyde, genipin, or a combination thereof.

7. An implantable surgical graft comprising the composition of any one of claims 1-6.

8. A decellularized nipple, areola, or nipple attached to an areola for use in grafting to the chest of a subject with Poland Syndrome, wherein at least one of the decellularized nipple, areola, or nipple attached to the areola when grafted onto the chest of the subject with Poland Syndrome promotes the regeneration and/or growth of at least one of the decellularized nipple, areola, or nipple attached to the areola, which is/are grafted onto the chest of a subject afflicted with Poland Syndrome, and further wherein the decellularized nipple, areola, or nipple attached to the areola is produced by:
obtaining a donor nipple, donor areola, or donor nipple attached to an areola; and
decellularizing the nipple, areola, or nipple attached to the areola to remove substantially or all cells, wherein at least one matrix molecule is retained in the nipple, areola, or
nipple attached to the areola, which matrix molecule is selected from the group consisting of laminin, elastin, fibronectin, and collagen; and further wherein exogenous cells are further seeded onto the nipple, areola, or nipple attached to the areola, and wherein said exogenous seeded cells comprise a combination of keratinocytes, melanocytes, and nerve cells.

9. The decellularized nipple, areola, or nipple attached to an areola, for use according to claim 8, wherein the exogenous seeded cells originate from a skin punch taken from the same subject as the decellularized dermis and/or decellularized epidermis, or the exogenous seeded cells originate from a skin punch obtained from a compatible donor.

10. The decellularized nipple, areola, or nipple attached to an areola, for use according to claim 8 or claim 9, wherein said nerve cells comprise neurospheres or neuronal cells.

11. The decellularized nipple, areola, or nipple attached to an areola, for use according to any one of claims 8-10, wherein the collagen comprises a Type I collagen, a Type III collagen, a Type IV collagen, a Type VI collagen, or a combination thereof.

## Patentansprüche

1. Zusammensetzung, die im Wesentlichen aus dezellularisierter Dermis und/oder dezellularisierter Epidermis besteht, wobei die Dermis und/oder Epidermis wenigstens ein Matrixmolekül enthält, das aus der Gruppe bestehend aus Laminin, Elastin, Fibronektin und Kollagen ausgewählt ist; und wobei die dezellularisierte Dermis und/oder die dezellularisierte Epidermis ferner exogen ausgesäte Zellen aufweist, die eine Kombination aus Keratinozyten, Melanozyten und Nervenzellen aufweisen.

2. Zusammensetzung nach Anspruch 1, wobei die exogen ausgesäten Zellen von einer Hautstanze stammen, die von demselben Subjekt wie die dezellularisierte Dermis und/oder dezellularisierte Epidermis erhalten wird, oder die exogen ausgesäten Zellen von einer Hautstanze stammen, die von einem kompatiblen Spender erhalten wird.

3. Zusammensetzung nach Anspruch 1 oder Anspruch 2, wobei die Dermis und/oder Epidermis eine dezellularisierte Brustwarze, einen dezellularisierten Warzenhof oder eine dezellularisierte Brustwarze, die an einem dezellularisierten Warzenhof befestigt ist, aufweist.

4. Zusammensetzung nach einem der vorhergehenden Ansprüche, wobei das Kollagen ein Typ-I-Kollagen, ein Typ-III-Kollagen, ein Typ-IV-Kollagen, ein Typ-VI-Kollagen oder eine Kombination davon aufweist.

5. Zusammensetzung nach einem der vorangehenden Ansprüche, ferner aufweisend ein Vernetzungsmittel.

6. Zusammensetzung nach Anspruch 5, wobei das Vernetzungsmittel Glutaraldehyd, Genipin oder eine Kombination davon aufweist.

7. Implantierbares chirurgisches Transplantat, aufweisend die Zusammensetzung nach einem der Ansprüche 1 bis 6.

8. Dezellularisierte Brustwarze, Warzenhof oder an einem Warzenhof befestigte Brustwarze zur Verwendung bei der Transplantation auf die Brust eines Subjekts mit Poland-Syndrom, wobei wenigstens eine der dezellularisierten Brustwarze, des Warzenhofs oder der am Warzenhof befestigten Brustwarze, wenn sie auf die Brust des Subjekts mit Poland-Syndrom transplantiert wird, die Regeneration und/oder das Wachstum von wenigstens einer der dezellularisierten Brustwarze, des Warzenhofs oder der am Warzenhof befestigten Brustwarze fördert, der/die auf die Brust eines mit dem Poland-Syndrom behafteten Subjekts transplantiert wird/werden, und wobei ferner die dezellularisierte Brustwarze, der Warzenhof oder die am Warzenhof befestigte Brustwarze hergestellt wird durch:
Erhalten einer Spenderbrustwarze, eines Spenderwarzenhofs oder einer an einem Warzenhof befestigten Spenderbrustwarze; und
Dezellularisieren der Brustwarze, des Warzenhofs oder der am Warzenhof befestigten Brustwarze, um im Wesentlichen oder alle Zellen zu entfernen,
wobei wenigstens ein Matrixmolekül in der Brustwarze, dem Warzenhof oder der am Warzenhof befestigten Brustwarze zurückgehalten wird, welches Matrixmolekül aus der Gruppe bestehend aus Laminin, Elastin, Fibronektin und Kollagen ausgewählt ist; und wobei ferner exogene Zellen ferner auf die Brustwarze, den Warzenhof oder die am Warzenhof befestigte Brustwarze ausgesät werden, und wobei die exogen ausgesäten Zellen eine Kombination von Keratinozyten, Melanozyten und Nervenzellen aufweisen.

9. Dezellularisierte Brustwarze, Warzenhof oder an einem Warzenhof befestigte Brustwarze zur Verwendung gemäß Anspruch 8, wobei die exogen ausgesäten Zellen von einer Hautstanze stammen, die von demselben Subjekt wie die dezellularisierte Dermis und/oder dezellularisierte Epidermis erhalten wird, oder die exogen ausgesäten Zellen von einer Hautstanze stammen, die von einem kompatiblen Spender erhalten wird.

10. Dezellularisierte Brustwarze, Warzenhof oder an einem Warzenhof befestigte Brustwarze zur Verwendung gemäß Anspruch 8 oder 9, wobei die Nervenzellen Neurosphären oder neuronale Zellen aufweisen.

11. Dezellularisierte Brustwarze, Warzenhof oder an einem Warzenhof befestigte Brustwarze zur Verwendung nach einem der Ansprüche 8 bis 10, wobei das Kollagen ein Typ-I-Kollagen, ein Typ-III-Kollagen, ein Typ-IV-Kollagen, ein Typ-VI-Kollagen oder eine Kombination davon aufweist.

## Revendications

1. Composition constituée essentiellement de derme décellularisé et/ou d'épiderme décellularisé, dans laquelle le derme et/ou l'épiderme retiennent au moins une molécule matricielle sélectionnée dans le groupe constitué de la laminine, de l'élastine, de la fibronectine et du collagène ; et dans laquelle le derme décellularisé et/ou l'épiderme décellularisé comprennent en outre des cellules exogènes ensemencées comprenant une combinaison de kératinocytes, de mélanocytes et de cellules nerveuses.

2. Composition selon la revendication 1, dans laquelle les cellules exogènes ensemencées proviennent d'un emporte-pièce de peau obtenu à partir du même sujet que le derme décellularisé et/ou l'épiderme décellularisé, ou dans laquelle les cellules exogènes ensemencées proviennent d'un emporte-pièce de peau obtenu à partir d'un donneur compatible.

3. Composition selon la revendication 1 ou 2, dans laquelle le derme et/ou l'épiderme comprennent un mamelon décellularisé, une aréole décellularisée ou un mamelon décellularisé rattaché à une aréole décellularisée.

4. Composition selon l'une quelconque des revendications précédentes, dans laquelle le collagène se compose d'un collagène de type I, d'un collagène de type III, d'un collagène de type IV, d'un collagène de type VI ou d'une combinaison de ceux-ci.

5. Composition selon l'une quelconque des revendications précédentes, comprenant en outre un agent de réticulation.

6. Composition selon la revendication 5, dans laquelle l'agent de réticulation comprend le glutaraldéhyde, la génipine ou une combinaison de ceux-ci.

7. Greffe chirurgicale implantable, comprenant la composition selon l'une quelconque des revendications 1 à 6.

8. Mamelon décellularisé ou aréole décellularisée ou mamelon décellularisé rattaché à une aréole, pour une utilisation dans une greffe sur la poitrine d'un sujet ayant le syndrome de Poland, dans lequel au moins l'un parmi le mamelon décellularisé, l'aréole décellularisée et le mamelon décellularisé rattaché à l'aréole favorise, lorsque qu'il est greffé sur la poitrine du sujet ayant le syndrome de Poland, la régénération et/ou la croissance d'au moins l'un parmi le mamelon décellularisé, l'aréole décellularisée et le mamelon décellularisé rattaché à l'aréole, quand il est greffé sur la poitrine d'un sujet atteint du syndrome de Poland ; et en outre dans lequel le mamelon décellularisé ou l'aréole décellularisée ou le mamelon décellularisé rattaché à l'aréole est produit en :
obtenant un mamelon de donneur, une aréole de donneur ou un mamelon de donneur rattaché à une aréole ; et
décellularisant le mamelon, l'aréole ou le mamelon rattaché à l'aréole, afin d'éliminer de manière significative ou en totalité des cellules, dans lequel au moins une molécule matricielle est retenue dans le mamelon, l'aréole ou le mamelon rattaché à l'aréole, laquelle molécule matricielle est sélectionnée dans le groupe constitué de la laminine, de l'élastine, de la fibronectine et du collagène ; et en outre dans lequel des cellules exogènes sont en outre ensemencées sur le mamelon, l'aréole ou le mamelon rattaché à l'aréole, et lesdites cellules exogènes ensemencées comprennent une combinaison de kératinocytes, de mélanocytes et de cellules nerveuses.

9. Mamelon décellularisé ou aréole décellularisée ou mamelon décellularisé rattaché à une aréole, pour une utilisation selon la revendication 8, dans lequel les cellules exogènes ensemencées proviennent d'un emporte-pièce de peau prélevé à partir du même sujet que le derme décellularisé et/ou l'épiderme décellularisé, ou dans lequel les cellules exogènes ensemencées proviennent d'un emporte-pièce de peau obtenu à partir d'un donneur compatible.

10. Mamelon décellularisé ou aréole décellularisée ou mamelon décellularisé rattaché à une aréole, pour une utilisation selon la revendication 8 ou 9, dans lequel lesdites cellules nerveuses comprennent des neurosphères ou des cellules neuronales.

11. Mamelon décellularisé ou aréole décellularisée ou mamelon décellularisé rattaché à une aréole, pour une utilisation selon l'une quelconque des revendications 8 à 10, dans lequel le collagène se compose d'un collagène de type I, d'un collagène de type III, d'un collagène de type IV, d'un collagène de type VI ou d'une combinaison de ceux-ci.
